(19) Europäisches Patentamt
European Patent Office
Office européen des brevets

(11) EP 2 170 964 B1

(12) **EUROPÄISCHE PATENTSCHRIFT**

(45) Veröffentlichungstag und Bekanntmachung des Hinweises auf die Patenterteilung:
**22.02.2017 Patentblatt 2017/08**

(21) Anmeldenummer: **08774585.7**

(22) Anmeldetag: **01.07.2008**

(51) Int Cl.:
***C08F 220/34*** *(2006.01)* ***A61K 8/81*** *(2006.01)*

(86) Internationale Anmeldenummer:
**PCT/EP2008/058439**

(87) Internationale Veröffentlichungsnummer:
**WO 2009/010385 (22.01.2009 Gazette 2009/04)**

(54) **HOCHKATIONISCHE COPOLYMERE AUF BASIS QUARTERNISIERTER STICKSTOFFHALTIGER MONOMERE**

HIGH-CATION COPOLYMERS BASED ON QUATERNIZED NITROGEN-CONTAINING MONOMERS

COPOLYMÈRES À CHARGE CATIONIQUE ÉLEVÉE, À BASE DE MONOMÈRES AZOTÉS QUATERNISÉS

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **17.07.2007 EP 07112639**

(43) Veröffentlichungstag der Anmeldung:
**07.04.2010 Patentblatt 2010/14**

(73) Patentinhaber: **BASF SE**
**67056 Ludwigshafen am Rhein (DE)**

(72) Erfinder:
- **MEIER, Nicole**
**68307 Mannheim (DE)**
- **GARCIA CASTRO, Ivette**
**67067 Ludwigshafen (DE)**
- **WOOD, Claudia**
**69469 Weinheim (DE)**
- **STAUB, Jessica**
**67454 Hassloch (DE)**

(74) Vertreter: **Reinhardt, Jürgen et al**
**BASF Personal Care and Nutrition GmbH**
**Postfach 13 01 64**
**40551 Düsseldorf (DE)**

(56) Entgegenhaltungen:
**WO-A-93/25595**
**WO-A-95/27759**
**WO-A-2006/097514**
**US-A- 4 058 491**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 170 964 B1

## Beschreibung

Die vorliegende Erfindung betrifft ein Copolymer mit hoher kationischer Ladung auf Basis quaternisierter stickstoffhaltiger Monomere, kosmetische oder pharmazeutische Mittel, die wenigstens ein solches Copolymer enthalten sowie weitere Verwendungen dieser Copolymere.

Kosmetisch und pharmazeutisch akzeptable wasserlösliche Polymere dienen beispielsweise in Seifen, Cremes und Lotionen als Formulierungsmittel, z. B. als Verdicker, Schaumstabilisator oder Wasserabsorbens oder auch dazu, die reizende Wirkung anderer Inhaltsstoffe abzumildern oder die dermale Applikation von Wirkstoffen zu verbessern. Ihre Aufgabe in der Haarkosmetik besteht darin, die Eigenschaften des Haares zu beeinflussen. In der Pharmazie dienen sie beispielsweise als Beschichtungsmittel oder Bindemittel für feste Arzneiformen.

Für die Haarkosmetik werden filmbildende Polymere mit ionischen Gruppen beispielsweise als Conditioner dazu eingesetzt, um die Trocken- und Nasskämmbarkeit, das Anfassgefühl, den Glanz und die Erscheinungsform des Haars zu verbessern sowie dem Haar antistatische Eigenschaften zu verleihen. Je nach Anwendungszweck werden dabei wasserlösliche Polymere mit kationischen oder anionischen Funktionalitäten eingesetzt. So weisen Polymere mit kationischen funktionellen Gruppen strukturell bedingt eine hohe Affinität zur negativ geladenen Oberfläche des Haares auf.

Schwierigkeiten bereitet oft die Bereitstellung von Produkten mit einem komplexen Eigenschaftsprofil. So besteht ein Bedarf an Polymeren für haarkosmetische Mittel, die zur Bildung im Wesentlichen glatter, klebfreier Filme befähigt sind, die dem Haar gleichzeitig gute sensorisch erfassbare Eigenschaften, wie Elastizität, einen angenehmen Griff, sowie antistatische Eigenschaften zu verleihen. Weiterhin sollen die Trocken- und Nasskämmbarkeit, das Anfassgefühl, der Glanz und die Erscheinungsform des Haars verbessert werden.

Sollen diese Polymere in Haarsprayformulierungen eingesetzt werden, so ist zudem eine gute Treibgasverträglichkeit, die Eignung für einen Einsatz in low-VOC-Formulierungen, eine gute Löslichkeit in Wasser oder wässrig/alkoholischen Lösungsmittelgemischen und eine gute Auswaschbarkeit erwünscht. Ebenso ist ein Build-up-Effekt möglichst gering zu halten.

In vielen Fällen lässt sich das gewünschte Eigenschaftsprofil nur durch Einsatz mehrerer kosmetisch aktiver Komponenten, beispielsweise mehrerer Polymere mit ionischen Gruppen erzielen. Dabei zeigt sich jedoch häufig eine Unverträglichkeit der verschiedenen Komponenten miteinander, was beispielsweise dazu führen kann, dass sich keine klaren Formulierungen mehr herstellen lassen. Der Einsatz mehrerer miteinander nicht ausreichend verträglicher Polyelektrolyte kann zu einem unerwünschten Aussalzen führen. So sind kationische Polymere insbesondere auch essentielle Bestandteile von Shampoos, die im allgemeinen auch anionische Tenside enthalten. In vielen Fällen führen Unverträglichkeiten zwischen den kationischen Polymeren und diesen anionischen Tensiden dann zu unzureichender Lagerstabilität der Zubereitungen.

WO 00/42985 beschreibt auf S. 19-20 zahlreiche kationische Polymere, die als Konditioniermittel in kosmetischen Zubereitungen geeignet sind. Genannt werden kommerziell erhältliche Polymere wie Jaguar®C-14-S, Jaguar®C-17, Jaguar®C-16, das Ucare Polymer JR-30M, JR-400, LR400, Catanal, Celquat, Merquat®100, Merquat®550, Merquat®S, Merquat®3330, Merquat®2001, Gafquat®755N, Luviquat®FC370, Polyquaternium 2, Polyalkylenimine, Aqualon®N-Hance.

Die Terpolymere der vorliegenden Erfindung werden allerdings nicht beschrieben.

DE 102 41 296 beschreibt Wasser-in-Wasser-Emulsionspolymerisate aus N-Vinylpyrrolidon und Quat-311 bzw. aus N-Vinylpyrrolidon und quaternisiertem N-Vinylimidazol, die als Konditionierungsmittel geeignet sind. Weiterhin nennt diese Schrift in Absatz [0191] zahlreiche weitere kationische Copolymere als in Shampoos übliche Konditioniermittel.

Die Terpolymere der vorliegenden Erfindung werden allerdings nicht beschrieben.

WO 2005/005497 beschreibt eine wässrige Polymerdispersion Pd), die erhältlich ist durch radikalische Polymerisation eines Monomergemischs M), enthaltend

a) wenigstens eine α,β-ethylenisch ungesättigte amidgruppenhaltige Verbindung der allgemeinen Formel I

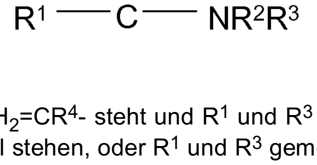

(I)

wobei
$R^2$ für eine Gruppe der Formel $CH_2$=$CR^4$- steht und $R^1$ und $R^3$ unabhängig voneinander für H, Alkyl, Cycloalkyl, Heterocycloalkyl, Aryl oder Hetaryl stehen, oder $R^1$ und $R^3$ gemeinsam mit der Amidgruppe, an die sie gebunden sind, für ein Lactam mit 5 bis 8 Ringatomen stehen,

b) wenigstens eine radikalisch polymerisierbare vernetzende Verbindung mit wenigstens zwei α,β-ethylenisch ungesättigten Doppelbindungen pro Molekül,

c) wenigstens eine Verbindung mit einer radikalisch polymerisierbaren $\alpha,\beta$-ethylenisch ungesättigten Doppelbindung und mindestens einer kationogenen und/oder kationischen Gruppe pro Molekül,

in einem wässrigen Medium in Gegenwart wenigstens eines polymeren anionischen Dispergiermittels D). Sie eignen sich als Konditioniermittel für kosmetische Zubereitungen, insbesondere Shampoos.

Copolymere, die sowohl Monomere des Typs a) als auch des Typs b) einpolymerisiert enthalten, werden nicht beschrieben.

WO 06/097514 betrifft die Verwendung eines wasserlöslichen oder wasserdispergierbaren vernetzten Polymeren erhältlich durch Polymerisation eines Gemisches umfassend 99,99 bis 10 Gew.-% wenigstens einer $\alpha,\beta$-ethylenisch ungesättigten Verbindung mit wenigstens einer kationogenen und/oder kationischen Gruppe pro Molekül, 0 bis 90 Gew.-% wenigstens einer von a) verschiedenen monoethylenisch ungesättigten a-midgruppenhaltigen Verbindung sowie 0,01 bis 5 Gew.-% eines Vernetzers zur Modifizierung der Rheologie wässriger, alkoholischer oder wässrig/alkoholischer Zusammensetzungen.

Copolymere, die sowohl Monomere des Typs a) als auch des Typs b) einpolymerisiert enthalten, werden nicht beschrieben.

Trotz der umfangreichen Bemühungen besteht nach wie vor Verbesserungsbedarf bei den aus dem Stand der Technik bekannten Polymere für Konditioniermittel und Shampoos. Gute Eigenschaften sind bezüglich der Konditionierung des Haars in seinen sensorisch erfassbaren Eigenschaften wie Griff, Volumen, Handhabbarkeit usw. gewünscht. Ferner sollen sich die Polymere durch eine gute Verträglichkeit mit anderen Formulierungsbestandteilen auszeichnen.

Überraschenderweise wurde gefunden, dass sich für die zuvor genannten Anforderungen besonders Copolymere eignen, die erhältlich sind durch Polymerisation von

a) wenigstens einem $\alpha,\beta$-ethylenisch ungesättigten Monomer a) der allgemeinen Formel I

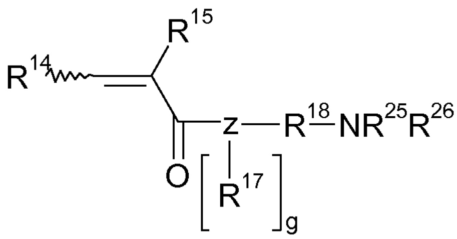 (I)

wobei

$R^{14}$ und $R^{15}$ unabhängig voneinander ausgewählt sind aus der Gruppe bestehend aus Wasserstoff, C1-C8 linear- oder verzweigtkettige Alkyl, Methoxy, E-thoxy, 2-Hydroxyethoxy, 2-Methoxyethoxy und 2-Ethoxyethyl,
$R^{17}$ Wasserstoff oder Methyl ist,
$R^{18}$ Alkylen oder Hydroxyalkylen mit 1 bis 24 C-Atomen, optional substituiert durch Alkyl, bevorzugt $C_2H_4$, $C_3H_6$, $C_4H_8$, $CH_2$-CH(OH)-$CH_2$ ist,
g 0 oder 1 ist,
Z Stickstoff wenn g = 1 oder Sauerstoff wenn g = 0 ist,
$R^{25}$ bzw. $R^{26}$ jeweils und unabhängig voneinander ausgewählt sind aus der Gruppe bestehend aus Wasserstoff, C1-C40 linear- oder verzweigtkettige Alkyl, Formyl, C1-C10 linear- oder verzweigtkettige Acyl, N,N-Dimethylaminoethyl, 2-Hydroxyethyl, 2-Methoxyethyl, 2-Ethoxyethyl, Hydroxypropyl, Methoxypropyl, Ethoxypropyl oder Benzyl,

wobei wenigstens 60 % der Stickstoffatome von a) quartäre Stickstoffatome sind,

b) wenigstens einem $\alpha,\beta$-ethylenisch ungesättigten Monomer b) der allgemeinen Formel II

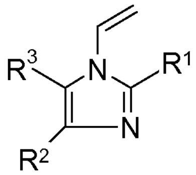 (II)

wobei $R^1$ bis $R^3$ unabhängig voneinander Wasserstoff, $C_1$-$C_4$-Alkyl oder Phenyl bedeuten;

c) wenigstens einem $\alpha,\beta$-ethylenisch ungesättigten Monomer c) der allgemeinen Formel III

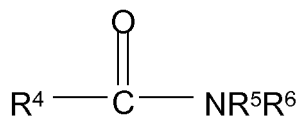 (III)

wobei

einer der Reste $R^4$ bis $R^6$ für eine Gruppe der Formel $CH_2=CR^7$- mit $R^7$ = H oder $C_1$-$C_4$-Alkyl steht und die übrigen Reste $R^4$ bis $R^6$ unabhängig voneinander für H, Alkyl, Cycloalkyl, Heterocycloalkyl, Aryl oder Hetaryl stehen,

wobei $R^4$ und $R^5$ gemeinsam mit der Amidgruppe, an die sie gebunden sind, auch für ein Lactam mit 5 bis 8 Ringatomen stehen können,

wobei $R^5$ und $R^6$ gemeinsam mit dem Stickstoffatom, an das sie gebunden sind, auch für einen fünf- bis siebengliedrigen Heterocyclus stehen können

d) gegebenenfalls wenigstens einem weiteren radikalisch polymerisierbaren, von a), b) und c) verschiedenen Monomer d).

Im Rahmen der vorliegenden Erfindung umfasst der Ausdruck Alkyl geradkettige und verzweigte Alkylgruppen. Geeignete kurzkettige Alkylgruppen sind z. B. geradkettige oder verzweigte $C_1$-$C_7$-Alkyl-, bevorzugt $C_1$-$C_6$-Alkyl- und besonders bevorzugt $C_1$-$C_4$-Alkylgruppen. Dazu zählen insbesondere Methyl, Ethyl, Propyl, Isopropyl, n-Butyl, Isobutyl, sec.-Butyl, tert.-Butyl, n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 1-Methylhexyl, 2-Methylhexyl, 2-Ethylpentyl, 1-Propylbutyl, Octyl etc.

Verzweigtes $C_3$-$C_5$-Alkyl steht vorzugsweise für Isopropyl, Isobutyl, sec.-Butyl, tert.-Butyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl. Bevorzugt ist tert.-Butyl.

Geeignete längerkettige $C_8$-$C_{30}$-Alkyl- bzw. $C_8$-$C_{30}$-Alkenylgruppen sind geradkettige und verzweigte Alkyl- bzw. Alkenylgruppen. Bevorzugt handelt es sich dabei um überwiegend lineare Alkylreste, wie sie auch in natürlichen oder synthetischen Fettsäuren und Fettalkoholen sowie Oxoalkoholen vorkommen, die gegebenenfalls zusätzlich einfach, zweifach oder mehrfach ungesättigt sein können. Dazu zählen z. B. n-Hexyl(en), n-Heptyl(en), n-Octyl(en), n-Nonyl(en), n-Decyl(en), n-Undecyl(en), n-Dodecyl(en), n-Tridecyl(en), n-Tetradecyl(en), n-Pentadecyl(en), n-Hexadecyl(en), n-Heptadecyl(en), n-Octadecyl(en), n-Nonadecyl(en), Arachinyl(en), Behenyl(en), Lignocerinyl(en), Melissinyl(en), etc.

Cycloalkyl steht vorzugsweise für $C_5$-$C_8$-Cycloalkyl, wie Cyclopentyl, Cyclohexyl, Cycloheptyl oder Cyclooctyl.

Aryl umfasst unsubstituierte und substituierte Arylgruppen und steht vorzugsweise für Phenyl, Tolyl, Xylyl, Mesityl, Naphthyl, Fluorenyl, Anthracenyl, Phenanthrenyl, Naphthacenyl und insbesondere für Phenyl, Tolyl, Xylyl oder Mesityl.

Im Folgenden werden Verbindungen, die sich von Acrylsäure und Methacrylsäure ableiten können teilweise verkürzt durch Einfügen der Silbe "(meth)" in die von der Acrylsäure abgeleitete Verbindung bezeichnet.

Unter wasserlöslichen Monomeren und Polymeren werden im Rahmen der vorliegenden Erfindung Monomere und Polymere verstanden, die sich zu mindestens 1 g/l bei 20 °C in Wasser lösen. Unter wasserdispergierbaren Monomeren und Polymeren werden Monomere und Polymere verstanden, die unter Anwendung von Scherkräften, beispielsweise durch Rühren, in dispergierbare Partikel zerfallen. Hydrophile Monomere sind vorzugsweise wasserlöslich oder zumindest wasserdispergierbar Die erfindungsgemäßen Copolymere weisen in einer speziellen Ausführung keine siliciumatomhaltigen Gruppen auf.

Erfindungsgemäß sind wenigstens 60, bevorzugt 75, weiter bevorzugt 90 und insbesondere wenigstens 95 % der Stickstoffatome von Monomer a) quartäre Stickstoffatome, insbesondere quartäre Ammoniumgruppen. Unter quartären Ammoniumgruppen versteht der Fachmann geladene kationische Gruppen, die sich aus Aminstickstoffatomen durch Quaternisierung mit Alkylierungsmitteln erzeugen lassen.

Geeignete Alkylierungsmittel sind $C_1$-$C_4$-Alkylhalogenide oder -sulfate, wie Ethylchlorid, Ethylbromid, Methylchlorid, Methylbromid, Dimethylsulfat und Diethylsulfat.

Ein bevorzugtes Quaternisierungsmittel ist Methylchlorid. Ein anderes bevorzugtes Quaternisierungsmittel ist Diethylsulfat.

Geladene kationische Gruppen (jedoch keine quartären Ammoniumgruppen im Sinne der Erfindung) lassen sich aus den Aminstickstoffen auch durch Protonierung mit Säuren erzeugen. Geeignete Säuren sind z. B. Carbonsäuren, wie Milchsäure, oder Mineralsäuren, wie Phosphorsäure, Schwefelsäure und Salzsäure.

Erfindungsgemäß besitzen also wenigstens 60, bevorzugt 75, weiter bevorzugt 90 und insbesondere wenigstens 95 mol-% von Monomer a) folgende Strukturformel:

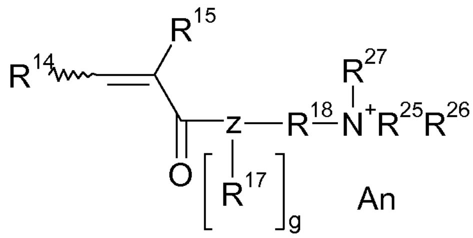 (V)

wobei $R^{27}$ H, $C_1$-$C_4$-Alkyl, bevorzugt Methyl oder Ethyl
und An Halogenion, bevorzugt $Cl^-$, $CH_3SO_4^-$, $C_2H_5SO_4^-$, $(SO_4^{2-})_{0,5}$

Monomer a)

Bevorzugte Monomere a) sind am Stickstoff ein- oder zweifach $C_1$-$C_{24}$-Alkyl substituierte Ester der (Meth)acrylsäure mit Aminoalkoholen. Besonders bevorzugt sind diese ausgewählt aus der Gruppe bestehend aus N-Methylaminoethyl(meth)acrylat, N-Ethylaminoethyl(meth)acrylat, N-(n-Propyl)aminoethyl(meth)acrylat, N-(n-Butyl)aminoethyl(meth)acrylat, N-(tert.-Butyl)aminoethyl(meth)acrylat, N,N-Dimethylaminomethyl(meth)acrylat, N,N-Dimethylaminoethyl(meth)acrylat, N,N-Diethylaminoethyl(meth)acrylat, N,N-Dimethylaminopropyl(meth)acrylat, N,N-Diethylaminopropyl(meth)acrylat und N,N-Dimethylaminocyclohexyl(meth)acrylat. Besonders bevorzugt ist N,N-Dimethylaminoethylmethacrylat.

Weitere bevorzugte Monomere a) sind am Stickstoff ein- oder zweifach $C_1$-$C_{24}$-Alkyl substituierte Amide der (Meth)acrylsäure mit Diaminen. Besonders bevorzugt sind diese ausgewählt aus der Gruppe bestehend aus N-[2-(dimethylamino)ethyl]acrylamid, N-[2-(dimethylamino)ethyl]methacrylamid, N-[3-(dimethylamino)propyl]acrylamid, N-[3-(dimethylamino)propyl]methacrylamid, N-[4-(dimethylamino)butyl]acrylamid, N-[4-(dimethylamino)-butyl]methacrylamid, N-[2-(diethylamino)ethyl]acrylamid, N-[4-(dimethylamino)cyclohexyl]acrylamid, N-[4-(dimethylamino)cyclohexyl]methacrylamid, N-[8-(Dimethylamino)octyl]methacrylamid, N-[12-(Dimethylamino)dodecyl]-methacrylamid, N-[3-(Diethylamino)propyl]methacrylamid und N-[3-(Diethylamino)propyl]acrylamid. Besonders bevorzugt ist N-[3-(dimethylamino)propyl]methacrylamid.

Erfindungsgemäß weisen wenigstens 60, bevorzugt 75, weiter bevorzugt 90 und insbesondere wenigstens 95 Mol-% von Monomer a), bezogen auf die Gesamtmenge aller Monomere a), wenigstens ein quartäres Stickstoffatom auf.

Bevorzugt ist wenigstens ein Monomer a) ausgewählt unter quaternisiertem N,N-Dimethylaminoethyl(meth)acrylat, quaternisiertem N-[3-(dimethylamino)propyl](meth)acrylamid und Mischungen davon.

Des Weiteren bevorzugt ist oder umfasst Monomer a) mit Methylchlorid, Dimethylsulfat oder Diethylsulfat quaternisiertes N,N-Dimethylaminoethyl(meth)acrylat.

Speziell ist oder umfasst Monomer a) mit Methylchlorid quaternisiertes N,N-Dimethylaminoethylmethacrylat.

Die erfindungsgemäßen Copolymere enthalten vorzugsweise wenigstens 60, weiter bevorzugt wenigstens 65 und insbesondere wenigstens 70 Gew.-%, bezogen auf das Gesamtgewicht der zur Polymerisation eingesetzten Monomere, wenigstens eines Monomers a) einpolymerisiert.

Die erfindungsgemäßen Copolymere enthalten vorzugsweise höchstens 90, weiter bevorzugt höchstens 85 und insbesondere höchstens 80 Gew.-%, bezogen auf das Gesamtgewicht der zur Polymerisation eingesetzten Monomere, wenigstens eines Monomers a) einpolymerisiert.

Monomer b)

Die erfindungsgemäßen Copolymere enthalten als Verbindung b) wenigstens ein $\alpha,\beta$-ethylenisch ungesättigtes Monomer b) der allgemeinen Formel II einpolymerisiert

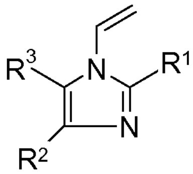

(II)

wobei $R^1$ bis $R^3$ unabhängig voneinander Wasserstoff, $C_1$-$C_4$-Alkyl oder Phenyl bedeuten.

Beispiele für geeignete Verbindungen der allgemeinen Formel (II) sind folgender Tabelle 1 zu entnehmen:

Tabelle 1

| $R^1$ | $R^2$ | $R^3$ |
|---|---|---|
| | | |
| H | H | H |
| Me | H | H |
| H | Me | H |
| H | H | Me |
| Me | Me | H |
| H | Me | Me |
| Me | H | Me |
| Ph | H | H |
| H | Ph | H |
| H | H | Ph |
| Ph | Me | H |
| Ph | H | Me |
| Me | Ph | H |
| H | Ph | Me |
| H | Me | Ph |
| Me | H | Ph |
| Me = Methyl<br>Ph = Phenyl | | |

Bevorzugt als Monomer b) ist 1-Vinylimidazol (N-Vinylimidazol) und sind Mischungen, die N-Vinylimidazol enthalten.

Die erfindungsgemäßen Copolymere enthalten vorzugsweise wenigstens 5, weiter bevorzugt wenigstens 7,5 und insbesondere wenigstens 10 Gew.-%, bezogen auf das Gesamtgewicht der zur Polymerisation eingesetzten Monomere, wenigstens eines Monomers b) einpolymerisiert.

Die erfindungsgemäßen Copolymere enthalten vorzugsweise höchstens 20, weiter bevorzugt höchstens 17,5 und insbesondere höchstens 15 Gew.-%, bezogen auf das Gesamtgewicht der zur Polymerisation eingesetzten Monomere, wenigstens eines Monomers b) einpolymerisiert.

In einer Ausführungsform der Erfindung können auch die Monomere b) kationische Ladungen tragen. Dabei trägt das an der Doppelbindung befindliche Stickstoffatom in der allgemeinen Formel II einen weiteren Substituenten, vorzugsweise Methyl oder Ethyl. Solche Monomere b) sind beispielsweise 3-Methyl-1-vinylimidazoliumchlorid, - methosulfat und -ethosulfat.

In einer bevorzugten Ausführungsform der Erfindung besitzen höchstens 20, weiter bevorzugt höchstens 10, besonders bevorzugt höchstens 5 und insbesondere höchstens 1 Mol-% der Monomere b) ein quartäres Stickstoffatom. Am meisten bevorzugt ist es, Monomer b) in nicht quaternisiertem Zustand zur Polymerisation einzusetzen.

Monomer c)

Bevorzugte Monomere c) sind offenkettige N-Vinylamidverbindungen wie N-Vinylformamid, N-Vinyl-N-methylformamid, N-Vinylacetamid, N-Vinyl-N-methylacetamid, N-Vinyl-N-ethylacetamid, N-Vinylpropionamid, N-Vinyl-N-methylpropionamid, N-Vinyl-butyramid und Mischungen davon.
Weitere bevorzugte Monomere c) sind ausgewählt aus der Gruppe bestehend aus Acrylamid, den N-Vinylderivaten von gegebenfalls alkylsubstituiertem 2-Pyrrolidon, gegebenfalls alkylsubstituiertem 2-Piperidon und gegebenfalls alkylsubstituiertem ε-Caprolactam.
Weiterhin bevorzugte Monomere c) sind ausgewählt aus der Gruppe bestehend aus den N-Vinylderivaten von 2-Pyrrolidon, 3-Methyl-2-pyrrolidon, 4-Methyl-2-pyrrolidon, 5-Methyl-2-pyrrolidon, 3-Ethyl-2-pyrrolidon, 3-Propyl-2-pyrrolidon, 3-Butyl-2-pyrrolidon, 3,3-Dimethyl-2-pyrrolidon, 3,5-Dimethyl-2-pyrrolidon, 5,5-Dimethyl-2-pyrrolidon, 3,3,5-Trimethyl-2-pyrrolidon, 5-Methyl-5-ethyl-2-pyrrolidon, 3,4,5-Trimethyl-2-pyrrolidon, 3-Methyl-2-piperidon, 4-Methyl-2-piperidon, 5-Methyl-2-piperidon, 6-Methyl-2-piperidon, 6-Ethyl-2-piperidon, 3,5-Dimethyl-2-piperidon, 4,4-Dimethyl-2-piperidon, 3-Methyl-ε-caprolactam, 4-Methyl-ε-caprolactam, 5-Methyl-ε-caprolactam, 6-Methyl-ε-caprolactam, 7-Methyl-ε-caprolactam, 3-Ethyl-ε-caprolactam, 3-Propyl-ε-caprolactam, 3-Butyl-ε-caprolactam, 3,3-Dimethyl-ε-caprolactam, 7,7-Dimethyl-ε-caprolactam und deren Mischungen.
Besonders bevorzugt ist oder umfasst Monomer c) N-Vinylpyrrolidon oder N-Vinylcaprolactam. Am meisten bevorzugt ist oder umfasst Monomer c) N-Vinylpyrrolidon.

Monomer d)

Die erfindungsgemäßen Copolymere enthalten gegebenenfalls wenigstens ein weiteres radikalisch polymerisierbares, von a), b) und c) verschiedenes Monomer d) einpolymerisiert.
Die erfindungsgemäßen Copolymere können zusätzlich wenigstens ein von den Komponenten a) bis d) verschiedenes, damit copolymerisierbares Monomer e) einpolymerisiert enthalten.
Monomer d) ist beispielsweise ausgewählt unter von a) bis c) verschiedenen Estern α,β-ethylenisch ungesättigter Mono- und Dicarbonsäuren mit $C_1$-$C_{30}$-Alkanolen und $C_1$-$C_{30}$-Alkandiolen, Amiden α,β-ethylenisch ungesättigter Mono- und Dicarbonsäuren mit $C_2$-$C_{30}$-Aminoalkoholen, die eine primäre oder sekundäre Aminogruppe aufweisen, Estern von Vinylalkohol und Allylalkohol mit $C_1$-$C_{30}$-Monocarbonsäuren, Vinylethern, Vinylaromaten, Vinylhalogeniden, Vinylidenhalogeniden, $C_1$-$C_8$-Monoolefinen, nicht aromatischen Kohlenwasserstoffen mit mindestens zwei konjugierten Doppelbindungen und Mischungen davon.
Geeignete Monomere d) sind weiterhin 2-Hydroxyethylacrylat, 2-Hydroxyethylmethacrylat, 2-Hydroxyethylethacrylat, 2-Hydroxypropylacrylat, 2-Hydroxypropylmethacrylat, 3-Hydroxypropylacrylat, 3-Hydroxypropylmethacrylat, 3-Hydroxybutylacrylat, 3-Hydroxybutylmethacrylat, 4-Hydroxybutylacrylat, 4-Hydroxybutylmethacrylat, 6-Hydroxyhexylacrylat, 6-Hydroxyhexylmethacrylat, 3-Hydroxy-2-ethylhexylacrylat und 3-Hydroxy-2-ethylhexylmethacrylat.
Geeignete zusätzliche Monomere d) sind weiterhin 2-Hydroxyethylacrylamid, 2-Hydroxyethylmethacrylamid, 2-Hydroxyethylethacrylamid, 2-Hydroxypropylacrylamid, 2-Hydroxypropylmethacrylamid, 3-Hydroxypropylacrylamid, 3-Hydroxypropylmethacrylamid, 3-Hydroxybutylacrylamid, 3-Hydroxybutylmethacrylamid, 4-Hydroxybutylacrylamid, 4-Hydroxybutylmethacrylamid, 6-Hydroxyhexylacrylamid, 6-Hydroxyhexylmethacrylamid, 3-Hydroxy-2-ethylhexylacrylamid und 3-Hydroxy-2-ethylhexylmethacrylamid.
Geeignete Monomere d) sind auch Polyetheracrylate, worunter im Rahmen dieser Erfindung allgemein Ester α,β-ethylenisch ungesättigter Mono- und Dicarbonsäuren mit Polyetherolen verstanden werden. Geeignete Polyetherole sind lineare oder verzweigte, endständige Hydroxylgruppen aufweisende Substanzen, die Etherbindungen enthalten. Im Allgemeinen weisen sie ein Molekulargewicht im Bereich von etwa 150 bis 20000 auf. Geeignete Polyetherole sind Polyalkylenglykole, wie Polyethylenglykole, Polypropylenglykole, Polytetrahydrofurane und Alkylenoxidcopolymere. Geeignete Alkylenoxide zur Herstellung von Alkylenoxidcopolymeren sind z. B. Ethylenoxid, Propylenoxid, Epichlorhydrin, 1,2- und 2,3-Butylenoxid. Die Alkylenoxidcopolymere können die Alkylenoxideinheiten statistisch verteilt oder in Form von Blöcken einpolymerisiert enthalten. Bevorzugt sind Ethylenoxid/Propylenoxid-Copolymere.
Geeignete Monomere d) sind auch Polyetheracrylate der allgemeinen Formel IV

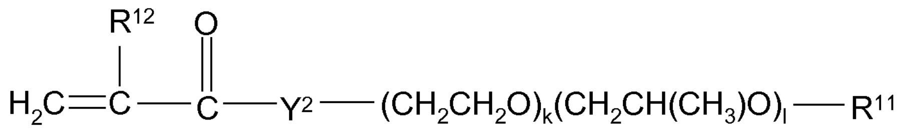

(IV)

worin

die Reihenfolge der Alkylenoxideinheiten beliebig ist,
k und l unabhängig voneinander für eine ganze Zahl von 0 bis 1000 stehen, wobei die Summe aus k und l mindestens 5 beträgt,
$R^{11}$ für Wasserstoff, $C_1$-$C_{30}$-Alkyl oder $C_5$-$C_8$-Cycloalkyl steht,
$R^{12}$ für Wasserstoff oder $C_1$-$C_8$-Alkyl steht,
$Y^2$ für O oder $NR^{13}$ steht, wobei $R^{13}$ für Wasserstoff, $C_1$-$C_{30}$-Alkyl oder $C_5$-$C_8$-Cycloalkyl steht. Bevorzugt steht k für eine ganze Zahl von 1 bis 500, insbesondere 3 bis 250. Bevorzugt steht l für eine ganze Zahl von 0 bis 100. Bevorzugt steht $R^{12}$ für Wasserstoff, Methyl, Ethyl, n-Propyl, Isopropyl, n-Butyl, sek.-Butyl, tert.-Butyl, n-Pentyl oder n-Hexyl, insbesondere für Wasserstoff, Methyl oder Ethyl. Vorzugsweise steht $R^{11}$ in der Formel II für Wasserstoff, Methyl, Ethyl, n-Propyl, Isopropyl, n-Butyl, sec-Butyl, n-Pentyl, n-Hexyl, Octyl, 2-Ethylhexyl, Decyl, Lauryl, Palmityl oder Stearyl. Vorzugsweise steht $Y^2$ in der Formel II für O oder NH.

Geeignete Polyetheracrylate d) sind z. B. die Polykondensationsprodukte der zuvor genannten $\alpha,\beta$-ethylenisch ungesättigten Mono- und/oder Dicarbonsäuren und deren Säurechloride, -amide und Anhydride mit Polyetherolen. Geeignete Polyetherole können leicht durch Umsetzung von Ethylenoxid, 1,2-Propylenoxid und/oder Epichlorhydrin mit einem Startermolekül, wie Wasser oder einem kurzkettigen Alkohol $R^{11}$-OH hergestellt werden. Die Alkylenoxide können einzeln, alternierend nacheinander oder als Mischung eingesetzt werden. Die Polyetheracrylate d) können allein oder in Mischungen zur Herstellung der erfindungsgemäß eingesetzten Polymere verwendet werden.

Geeignete zusätzliche Monomere d) sind Methyl(meth)acrylat, Methylethacrylat, Ethyl(meth)acrylat, Ethylethacrylat, n-Butyl(meth)acrylat, tert.-Butylmethacrylat, tert.-Butylethacrylat, n-Octyl(meth)acrylat, 1,1,3,3-Tetramethylbutyl(meth)acrylat, Ethylhexyl(meth)acrylat, n-Nonyl(meth)acrylat, n-Decyl(meth)acrylat, n-Undecyl(meth)acrylat, Tridecyl(meth)acrylat, Myristyl(meth)acrylat, Pentadecyl(meth)acrylat, Palmityl(meth)acrylat, Heptadecyl(meth)acrylat, Nonadecyl(meth)acrylat, Arrachinyl(meth)acrylat, Behenyl(meth)acrylat, Lignocerenyl(meth)acrylat, Cerotinyl(meth)acrylat, Melissinyl(meth)acrylat, Palmitoleinyl(meth)acrylat, Oleyl(meth)acrylat, Linolyl(meth)acrylat, Linolenyl(meth)acrylat, Stearyl(meth)acrylat, Lauryl(meth)acrylat und Mischungen davon. Bevorzugte Monomere e) sind die Ester $\alpha,\beta$-ethylenisch ungesättigter Mono- und Dicarbonsäuren mit $C_1$-$C_4$-Alkanolen.

Geeignete zusätzliche Monomere d) sind weiterhin Vinylacetat, Vinylpropionat, Vinylbutyrat und Mischungen davon.

Geeignete zusätzliche Monomere d) sind weiterhin Ethylen, Propylen, Isobutylen, Butadien, Styrol, $\alpha$-Methylstyrol, Acrylnitril, Methacrylnitril, Vinylchlorid, Vinylidenchlorid, Vinylfluorid, Vinylidenfluorid und Mischungen davon.

Bevorzugte Monomere d) sind Verbindungen mit einer radikalisch polymerisierbaren, $\alpha,\beta$-ethylenisch ungesättigten Doppelbindung und wenigstens einer anionogenen und/oder anionischen Gruppe pro Molekül.

Vorzugsweise umfasst die Komponente d) wenigstens eine Verbindung, die ausgewählt ist unter monoethylenisch ungesättigten Carbonsäuren, Sulfonsäuren, Phosphonsäuren und Mischungen davon. Zu den Monomeren d) zählen monoethylenisch ungesättigte Mono- und Dicarbonsäuren mit 3 bis 25 vorzugsweise 3 bis 6 C-Atomen, die auch in Form ihrer Salze oder Anhydride eingesetzt werden können. Beispiele hierfür sind Acrylsäure, Methacrylsäure, Ethacrylsäure, $\alpha$-Chloracrylsäure, Crotonsäure, Maleinsäure, Maleinsäureanhydrid, Itaconsäure, Citraconsäure, Mesaconsäure, Glutaconsäure, Aconitsäure und Fumarsäure. Zu den Monomeren d) zählen weiterhin die Halbester von monoethylenisch ungesättigten Dicarbonsäuren mit 4 bis 10 vorzugsweise 4 bis 6 C-Atomen, z. B. von Maleinsäure wie Maleinsäuremonomethylester. Zu den Monomeren d) zählen auch monoethylenisch ungesättigte Sulfonsäuren und Phosphonsäuren, beispielsweise Vinylsulfonsäure, Allylsulfonsäure, Sulfoethylacrylat, Sulfoethylmethacrylat, Sulfopropylacrylat, Sulfopropylmethacrylat, 2-Hydroxy-3-acryloxypropylsulfonsäure, 2-Hydroxy-3-methacryloxypropylsulfonsäure, Styrolsulfonsäure, 2-Acrylamido-2-methylpropansulfonsäure, Vinylphosphonsäure und Allylphosphonsäure. Zu den Monomeren d) zählen auch die Salze der zuvor genannten Säuren, insbesondere die Natrium-, Kalium- und Ammoniumsalze sowie die Salze mit Aminen. Die Monomere d) können als solche oder als Mischungen untereinander eingesetzt werden. Die angegebenen Gewichtsanteile beziehen sich sämtlich auf die Säureform.

Vorzugsweise ist oder umfasst d) wenigstens eine Verbindung, die ausgewählt ist unter Acrylsäure, Methacrylsäure, Ethacrylsäure, α-Chloracrylsäure, Crotonsäure, Maleinsäure, Maleinsäureanhydrid, Fumarsäure, Itaconsäure, Citraconsäure, Mesaconsäure, Glutaconsäure, Aconitsäure, 2-Acrylamido-2-methylpropansulfonsäure, Vinylphosphonsäure und Mischungen davon.

Besonders bevorzugt ist oder umfasst d) wenigstens eine Verbindung, die ausgewählt ist unter Acrylsäure, Methacrylsäure und Mischungen davon. In einer speziellen Ausführung ist oder umfasst d) Methacrylsäure.

Die zuvor genannten zusätzlichen Monomere d) können einzeln oder in Form von beliebigen Mischungen eingesetzt werden.

Die Menge der zur Polymerisation eingesetzten Monomere d) beträgt höchstens 20, bevorzugt höchstens 15 und besonders bevorzugt höchstens 10 Gew.-%, jeweils bezogen auf die Gesamtmenge aller zur Polymerisation eingesetzten Monomere.

Die Menge der zur Polymerisation eingesetzten Monomere d) beträgt wenigstens 0, bevorzugt wenigstens 3 und besonders bevorzugt wenigstens 5 Gew.-%, jeweils bezogen auf die Gesamtmenge aller zur Polymerisation eingesetzten Monomere.

Vernetzer e)

Die erfindungsgemäßen Copolymere können gewünschtenfalls wenigstens einen Vernetzer, d. h. eine Verbindung mit zwei oder mehr als zwei ethylenisch ungesättigten, nichtkonjugierten Doppelbindungen einpolymerisiert enthalten.

Vorzugsweise werden Vernetzer in einer Menge von 0,01 bis 2 Gew.-%, besonders bevorzugt 0,1 bis 1 Gew.-%, bezogen auf das Gesamtgewicht der zur Polymerisation eingesetzten Monomere, verwendet.

Geeignete Vernetzer e) sind zum Beispiel Acrylester, Methacrylester, Allylether oder Vinylether von mindestens zweiwertigen Alkoholen. Die OH-Gruppen der zugrunde liegenden Alkohole können dabei ganz oder teilweise verethert oder verestert sein; die Vernetzer enthalten aber mindestens zwei ethylenisch ungesättigte Gruppen.

Beispiele für die zugrunde liegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 1,4-Bis(hydroxymethyl)cyclohexan, Hydroxypivalinsäure-neopentylglykolmonoester, 2,2-Bis(4-hydroxyphenyl)-propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thio-pentan-1,5-diol, sowie Polyethylenglykole, Polypropylenglykole und Polytetrahydrofurane mit Molekulargewichten von jeweils 200 bis 10000. Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zugrunde liegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Vernetzer e) sind die Vinylester oder die Ester einwertiger, ungesättigter Alkohole mit ethylenisch ungesättigten $C_3$-$C_6$-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellithsäure, Phthalsäure, Terephthalsäure, Zitronensäure oder Bernsteinsäure.

Weitere geeignete Vernetzer e) sind Ester ungesättigter Carbonsäuren mit den oben beschriebenen mehrwertigen Alkoholen, beispielsweise der Ölsäure, Crotonsäure, Zimtsäure oder 10-Undecensäure.

Weitere geeignete Vernetzer e) sind Urethandiacrylate und Urethanpolyacrylate, wie sie z. B. unter der Bezeichnung Laromer® kommerziell erhältlich sind.

Geeignet als Vernetzer e) sind außerdem geradkettige oder verzweigte, lineare oder cyclische, aliphatische oder aromatische Kohlenwasserstoffe, die über mindestens zwei Doppelbindungen verfügen, die bei aliphatischen Kohlenwasserstoffen nicht konjugiert sein dürfen, z. B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20000.

Als Vernetzer e) sind ferner geeignet die Acrylsäureamide, Methacrylsäureamide und N-Allylamine von mindestens zweiwertigen Aminen. Solche Amine sind zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren, wie Acrylsäure, Methacrylsäure,

Itaconsäure, Maleinsäure, oder mindestens zweiwertigen Carbonsäuren, wie sie oben beschrieben wurden.

Ferner sind Triallylamin und Triallylmonoalkylammoniumsalze, z. B. Triallylmethylammoniumchlorid oder -methylsulfat, als Vernetzer e) geeignet.

Geeignet sind auch N-Vinyl-Verbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z. B. N,N'-Divinylethylenharnstoff oder N,N'-Divinylpropylenharnstoff.

Weitere geeignete Vernetzer e) sind Divinyldioxan, Tetraallylsilan oder Tetravinylsilan.

Selbstverständlich können auch Mischungen der vorgenannten Verbindungen e) eingesetzt werden.

Besonders bevorzugt eingesetzte Vernetzer e) sind beispielsweise Methylenbisacrylamid, Triallylamin und Triallylalkylammoniumsalze, Divinylimidazol, Pentaerythrittriallylether, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind.

Ganz besonders bevorzugt als Vernetzer e) sind Pentaerythrittriallylether, Methylenbisacrylamid, N,N'-Divinylethylenharnstoff, Triallylamin und Triallylmonoalkylammoniumsalze und Acrylsäureester von Glykol, Butandiol, Trimethylolpropan oder Glycerin oder Acrylsäureester von mit Ethylenoxid und/oder Epichlorhydrin umgesetztem Glykol, Butandiol, Trimethylolpropan oder Glycerin.

Lösungspolymerisation

Bevorzugt werden die Polymere durch Lösungspolymerisation in wässriger Lösung hergestellt. In einer bevorzugten Ausführungsform der Erfindung umfasst das Lösungsmittel Wasser und Alkohol.

Geeignete Alkohole sind beispielsweise Methanol, Ethanol, n-Propanol, Isopropanol, n-Butanol, sek.-Butanol, tert.-Butanol, 3-Methyl-1-butanol (Isoamylalkohol), n-Hexanol, Cyclohexanol oder Glykole wie Ethylenglykol, Propylenglykol und Butylenglykol sowie Alkylether mehrwertiger Alkohole wie Diethylenglykol, Triethylenglykol, Polyethylenglykolen mit zahlenmittleren Molekulargewichten bis etwa 3000, Glycerin.

Besonders bevorzugt ist oder umfasst der Alkohol Ethanol und/oder Isopropanol, insbesondere Isopropanol. Der Alkoholanteil an der gesamten Lösungsmittelmenge liegt im Bereich von 0 bis 50 Gew.-%, bevorzugt von 5 bis 40 Gew.-%, besonders bevorzugt von 10 bis 30 Gew.-%, jeweils bezogen auf die Gesamtmenge des Lösungsmittels.

Zusätzlich zu Alkohol und Wasser können in der Polymerisationslösung weitere Lösungsmittel enthalten sein. Prinzipiell kommen alle für die radikalische Polymerisation geeigneten Lösungsmittel in Frage wie beispielsweise Aceton, Acetonitril, Anilin, Anisol, Benzonitril, tert-Butylmethylether (TBME), Gamma-Butyrolacton, Chinolin, Chloroform, Cyclohexan, Diethylether, Dimethylacetamid, Dimethylformamid, Dimethylsulfoxid, Dioxan, Essigsäureethylester, Ethylendichlorid, Ethylenglycoldimethylether, Formamid, Hexan, Methylenchlorid, Methylethylketon, N-Methylformamid, Petrolether/Leichtbenzin, Propylencarbonat (4-Methyl-1,3-dioxol-2-on), Sulfolan, Tetrachlorethen, Tetrachlorkohlenstoff, Tetrahydrofuran, Toluol, 1,1,1-Trichlorethan, Trichlorethen, Triethylenglycoldimethylether (Triglyme) und deren Mischungen.

Unter Polymerisationslösung wird diejenige Substanzmischung verstanden, die nach Zugabe aller Komponenten und Beendigung der Polymerisation und vor dem ersten Aufarbeitungsschritt wie beispielsweise einer Trocknung, einer Neutralisation oder einer Wasserdampfdestillation vorliegt.

Die Menge an Wasser liegt bevorzugt im Bereich von 100 bis 50, besonders bevorzugt im Bereich von 90 bis 60 Gew.-%, bezogen auf das Lösungsmittel.

Die Menge an Alkohol liegt bevorzugt im Bereich von 0 bis 50, besonders bevorzugt im Bereich von 10 bis 40 Gew.-%, bezogen auf das Lösungsmittel.

Die Menge an weiterhin in der Polymerisationslösung enthaltenen Substanzen, welche im wesentlichen die Monomere a) bis d), der Initiator und gegebenenfalls Regler und Vernetzer sind, beträgt bevorzugt wenigstens 5, besonders bevorzugt wenigstens 10 und insbesondere wenigstens 20 und bevorzugt höchstens 55, besonders bevorzugt höchstens 50 und insbesondere höchstens 45 Gew.-% der Polymerisationslösung. Diese Menge kann auch als Feststoffgehalt der Polymerisationslösung bezeichnet werden.

Bevorzugt ist ein erfindungsgemäßes Verfahren bei dem die Temperatur der Polymerisationslösung im Bereich von 30°C bis 120°C, besonders bevorzugt im Bereich von 50°C bis 110°C und insbesondere im Bereich von 70°C bis 100°C liegt.

Die Polymerisation erfolgt üblicherweise unter atmosphärischem Druck, sie kann jedoch auch unter vermindertem oder erhöhtem Druck ablaufen. Ein geeigneter Druckbereich liegt zwischen 1 und 10 bar.

Als Initiator für die radikalische Polymerisation wird bevorzugt wenigstens ein wasserlöslicher Polymerisationsinitiator ausgewählt aus der Gruppe bestehend aus Peroxiden, Hydroperoxiden, Peroxodisulfaten, Percarbonaten, Peroxidestern, Azoverbindungen und deren Mischungen verwendet.

Unter einem wasserlöslichen Polymerisationsinitiator wird ein Initiator verstanden, der bei 20°C und 1013 mbar

zu wenigstens 1 g, bevorzugt zu wenigstens 10g in 1 Liter Wasser löslich ist.

In einer bevorzugten Ausführungsform der Erfindung wird der wasserlösliche Polymerisationsinitiator ausgewählt aus der Gruppe bestehend aus wasserlöslichen Azoverbindungen, Wasserstoffperoxid, Lithiumperoxodisulfat, Natriumperoxodisulfat, Kaliumperoxodisulfat, Ammoniumperoxodisulfat und deren Mischungen.

Weiterhin bevorzugt wird der wasserlösliche Polymerisationsinitiator ausgewählt aus der Gruppe bestehend aus 2,2'-Azobis[2-(5-methyl-2-imidazolin-2-yl)propan]dihydrochlorid 2,2'-Azobis[2-(2-imidazolin-2-yl)propan] dihydrochlorid 2,2'-Azobis[2-(2-imidazolin-2-yl)propandisulfatdihydrat 2,2'-Azobis(2-methylpropionamid)dihydrochlorid 2,2'-Azobis[N-(2-carboxyethyl)-2-methylpropionamidin]tetrahydrat 2,2'-Azobis[2-(3,4,5,6-tetrahydropyrimidin-2-yl)propan] dihydrochlorid 2,2'-Azobis{2-[1-(2-hydroxyethyl)-2-imidazolin-2-yl]propan}dihydrochlorid 2,2'-Azobis[2-(2-imidazolin-2-yl)propan] 2,2'-Azobis{2-methyl-N-[1,1-bis(hydroxymethyl)-2-hydroxyethyl]propionamid 2,2'-Azobis{2-methyl-N-[2-(1-hydroxybutyl)]propionamid} 2,2'-Azobis[2-methyl-N-(2-hydroxyethyl)propionamid] und deren Mischungen.

Auch wasserlösliche Redox-Initiatorsysteme können als Polymerisationsinitiatoren verwendet werden. Solche Redox-Initiatorsysteme enthalten mindestens eine peroxidhaltige Verbindung in Kombination mit einem Redox-Coinitiator z.B. reduzierend wirkende Schwefelverbindungen, beispielsweise Bisulfite, Sulfite, Thiosulfate, Dithionite und Tetrathionate von Alkalimetallen und Ammoniumverbindungen. So kann man Kombinationen von Peroxodisulfaten mit Alkalimetall- oder Ammoniumhydrogensulfiten einsetzen, z.B. Ammoniumperoxodisulfat und Ammoniumdisulfit. Die Menge der peroxidhaltigen Verbindung zum Redox-Coinitiator liegt im Bereich von 30:1 bis 0,05:1.

In Kombination mit den Initiatoren bzw. den Redoxinitiatorsystemen können zusätzlich Übergangsmetallkatalysatoren eingesetzt werden, z.B. Salze von Eisen, Kobalt, Nickel, Kupfer, Vanadium und Mangan. Geeignete Salze sind z.B. Eisen (II)sulfat, Kobalt (II)chlorid, Nickel(II)sulfat, oder Kupfer(I)chlorid. Bezogen auf die Monomeren wird das reduzierend wirkende Übergangsmetallsalz in einer Konzentration von 0,1 ppm bis 1000 ppm eingesetzt. So kann man Kombinationen von Wasserstoffperoxid mit Eisen(II)-Salzen einsetzen, wie beispielsweise 0,5 bis 30 % Wasserstoffperoxid und 0,1 bis 500 ppm Mohrsches Salz.

Weiterhin können in Kombination mit den oben genannten Initiatoren Redox-Coinitiatoren und/oder Übergangsmetallkatalysatoren mitverwendet werden, z.B. Benzoin, Dimethylanilin, Ascorbinsäure sowie Komplexe von Schwermetallen, wie Kupfer, Cobalt, Eisen, Mangan, Nickel und Chrom. Die üblicherweise verwendeten Mengen an Redox-Coinitiatoren bzw. Übergangsmetallkatalysatoren betragen etwa 0,1 bis 1000 ppm, bezogen auf die eingesetzten Mengen an Monomeren. Weitere geeignete Initiatoren sind beschrieben in den Kapiteln 20 und 21 von Macromolecules, Vol. 2, 2nd Ed., H. G. Elias, Plenum Press, 1984, New York, worauf hier vollumfänglich Bezug genommen wird. Weiterhin sind geeignete Photoinitiatoren beschrieben in S. P. Pappas, J. Rad. Cur., July 1987, S.6, worauf hier vollumfänglich Bezug genommen wird.

Die Menge des wenigstens einen für die Polymerisation der Monomeren eingesetzten wasserlöslichen Initiators beträgt vorzugsweise von 0,0001 bis 10, besonders bevorzugt 0,001 bis 5 und insbesondere 0,02 bis 3 Gew.-%, bezogen auf die Gesamtmenge der eingesetzten Monomere.

Zur Einstellung des Molekulargewichts kann die Polymerisation in Gegenwart wenigstens eines Reglers erfolgen. Als Regler können die üblichen, dem Fachmann bekannten Verbindungen, wie z. B. Schwefelverbindungen, z. B. Mercaptoethanol, 2-Ethylhexylthioglycolat, Thioglycolsäure oder Dodecylmercaptan sowie Tribromchlormethan oder andere Verbindungen, die regelnd auf das Molekulargewicht der erhaltenen Polymerisate wirken, eingesetzt werden. Ein bevorzugter Regler ist Cystein.

Die Lösungspolymerisation kann sowohl als Batchprozess als auch in Form eines Zulaufverfahrens, einschließlich Monomerenzulauf, Stufen- und Gradientenfahrweise, durchgeführt werden. Bevorzugt ist im Allgemeinen das Zulaufverfahren, bei dem man gegebenenfalls einen Teil des Polymerisationsansatzes vorlegt, auf die Polymerisationstemperatur erhitzt und anschließend den Rest des Polymerisationsansatzes, üblicherweise über einen oder auch mehrere, räumliche getrennte Zuläufe, kontinuierlich, stufenweise oder unter Überlagerung eines Konzentrationsgefälles unter Aufrechterhaltung der Polymerisation der Polymerisationszone zuführt.

In einer Ausführungsform der Erfindung wird die Gesamtmenge der Monomere a) bis d) und gegebenenfalls der Vernetzer e) und der Regler vorgelegt und der Initiator allmählich zur Reaktionsmischung zugegeben.

In einer weiteren Ausführungsform der Erfindung wird ein Teil von Monomer a), beispielsweise 1 bis 50, bevorzugt 5 bis 40, besonders bevorzugt 10 bis 30 Gew.-% von Monomer a), Lösungsmittel sowie ein Teil des gegebenenfalls verwendeten Reglers, beispielsweise 1 bis 50, bevorzugt 5 bis 40, besonders bevorzugt 10 bis 30 Gew.-% des Reglers vorgelegt und die übrigen Monomere b), c), gegebenenfalls d), Lösungsmittel und der restliche Regler in einem Zulauf sowie Lösungsmittel und Initiator in einem zweiten Zulauf allmählich zur Reaktionsmischung zugegeben. Dabei ist es vorteilhaft, sowohl in der Vorlage als auch in den Zuläufen Lösungsmittel zu verwenden.

In einer weiteren Ausführungsform der Erfindung wird der größte Teil von Monomer a), beispielsweise mehr als 50, bevorzugt mehr als 60, weiter bevorzugt mehr als 70, besonders bevorzugt mehr als 80 und insbesondere mehr als 90 Gew.-% vorgelegt und gemeinsam mit Lösungsmittel gegebenenfalls erwärmt. Dann wird ein Teil der Monomere b) und c), beispielsweise bis zu 50, bevorzugt bis zu 40, besonders bevorzugt bis zu 30 und insbesondere bis zu 20 Gew.-% der Monomere b) und c) zur gegebenenfalls erwärmten Vorlage gegeben und die Polymerisation mit Hilfe von

Initiator gestartet. Die Restmenge der Monomeren und des Initiators wird dann allmählich zudosiert.

In einer bevorzugten Ausführungsform werden die Monomere b) und c) als Mischung gemeinsam zudosiert.

Die Polymerisation wird bevorzugt unter weitgehendem Ausschluss von Sauerstoff ausgeführt. Bevorzugt ist es, die Polymerisation unter Schutzgasatmosphäre wie beispielsweise Argon- oder bevorzugt Stickstoffatmosphäre durchzuführen.

Die Polymerisation kann prinzipiell bei dem durch die eingesetzten Monomere resultierenden pH-Wert erfolgen.

Vorzugsweise wird der pH-Wert der Polymerisationslösung auf einen Wert von 5 bis 10, weiter bevorzugt 6 bis 9, besonders bevorzugt 6,5 bis 8,5 eingestellt. Vorzugsweise wird auch der pH-Wert der Vorlage und der verschiedenen Zuläufe auf einen Wert von 5 bis 10, weiter bevorzugt 6 bis 9, besonders bevorzugt 6,5 bis 8,5 eingestellt.

Es ist weiterhin vorteilhaft, den pH-Wert während der Polymerisation dann in diesem Bereich zu halten. Zur Einstellung des pH-Werts vor, während oder nach der Polymerisation eignen sich prinzipiell alle anorganischen oder organischen Basen (und gegebenenfalls Säuren), insbesondere solche, die außer einer etwaigen Salzbildung keine Reaktion mit den Monomeren eingehen. Geeignete Basen sind z. B. Alkali- und Erdalkalihydroxide, tertiäre Amine, wie Triethylamin, sowie Aminoalkohole, wie Triethanolamin, Methyldiethanolamin oder Dimethylethanolamin. Bevorzugt wird zur Einstellung des pH-Werts NaOH oder wenigstens ein tertiäres Amin, das insbesondere ausgewählt ist unter N,N-Dimethylethanolamin, N-Methyldiethanolamin, Triethanolamin und Mischungen davon, eingesetzt.

Zur Erzielung möglichst reiner Polymere mit geringem Restmonomergehalt kann sich an die Polymerisation (Hauptpolymerisation) ein Nachpolymerisationsschritt anschließen. Die Nachpolymerisation kann in Gegenwart desselben oder eines anderen Initiatorsystems wie die Hauptpolymerisation erfolgen. Vorzugsweise erfolgt die Nachpolymerisation mindestens bei der gleichen, vorzugsweise bei einer höheren Temperatur als die Hauptpolymerisation. Gewünschtenfalls kann der Reaktionsansatz im Anschluss an die Polymerisation oder zwischen dem ersten und dem zweiten Polymerisationsschritt einem Strippen mit Wasserdampf oder einer Wasserdampf-Destillation unterzogen werden, was insbesondere vorteilhaft zur Beseitigung von Komponenten mit unerwünschtem Geruch durchgeführt wird.

Die zur Polymerisation eingesetzten Monomeren werden vorzugsweise zu wenigstens 95, besonders bevorzugt zu wenigstens 99 und insbesondere zu wenigstens 99,9% umgesetzt (Polymerisationsgrad).

Die nach der Polymerisation in Lösung oder dispergiert vorliegenden Polymere können durch übliche, dem Fachmann bekannte Trocknungsverfahren in Pulver überführt werden. Bevorzugte Verfahren sind die Sprühtrocknung, die Sprühwirbelschichttrocknung, die Walzentrocknung und die Bandtrocknung. Ebenfalls anwendbar sind die Gefriertrocknung und die Gefrierkonzentrierung.

Gewünschtenfalls können Lösungsmittel auch durch übliche Methoden, z. B. Destillation bei verringertem Druck, teilweise oder vollständig entfernt werden.

Eine bevorzugte Ausführungsform der Erfindung sind Copolymere, die

a) mit Methylchlorid quaternisiertes Dimethylaminoethylmethacrylat
b) N-Vinylimidazol
c) Vinylpyrrolidon und/oder Vinylcaprolactam,
d) gegebenfalls Methacrylsäure,

einpolymerisiert enthalten.

Eine andere bevorzugte Ausführungsform der Erfindung sind Copolymere, die

e) mit Dimethylsulfat quaternisiertes Dimethylaminoethylmethacrylat
f) N-Vinylimidazol
g) Vinylpyrrolidon und/oder Vinylcaprolactam,
h) gegebenfalls Methacrylsäure,

einpolymerisiert enthalten.

In einer besonders bevorzugten Ausführungsform der Erfindung werden zur Herstellung der zuvor genannten Copolymere Monomere a) eingesetzt, die zu wenigstens 90 mol-%, bevorzugt zu wenigstens 99 mol-% quaternisiert sind.

Ein weiterer Gegenstand der Erfindung sind kosmetische oder pharmazeutische Mittel, enthaltend

A) wenigstens ein Copolymer, wie zuvor definiert, und
B) wenigstens einen kosmetisch akzeptablen Träger.

Die erfindungsgemäßen Mittel weisen vorzugsweise einen kosmetisch oder pharmazeutisch akzeptablen Träger B) auf, der ausgewählt ist unter

i) Wasser,

ii) wassermischbaren organischen Lösungsmitteln, vorzugsweise $C_2$-$C_4$-Alkanolen, insbesondere Ethanol,
iii) Ölen, Fetten, Wachsen,
iv) von iii) verschiedenen Estern von $C_6$-$C_{30}$-Monocarbonsäuren mit ein-, zwei- oder dreiwertigen Alkoholen,
v) gesättigten acyclischen und cyclischen Kohlenwasserstoffen,
vi) Fettsäuren,
vii) Fettalkoholen,
viii) Treibgasen,

und Mischungen davon.

Die erfindungsgemäßen Mittel weisen z. B. eine Öl- bzw. Fettkomponente B) auf, die ausgewählt ist unter: Kohlenwasserstoffen geringer Polarität, wie Mineralölen; linearen gesättigten Kohlenwasserstoffen, vorzugsweise mit mehr als 8 C-Atomen, wie Tetradecan, Hexadecan, Octadecan etc.; cyclischen Kohlenwasserstoffen, wie Decahydronaphthalin; verzweigten Kohlenwasserstoffen; tierischen und pflanzlichen Ölen; Wachsen; Wachsestern; Vaselin; Estern, bevorzugt Estern von Fettsäuren, wie z. B. die Ester von $C_1$-$C_{24}$-Monoalkoholen mit $C_1$-$C_{22}$-Monocarbonsäuren, wie Isopropylisostearat, n-Propylmyristat, iso-Propylmyristat, n-Propylpalmitat, iso-Propylpalmitat, Hexacosanylpalmitat, Octacosanylpalmitat, Triacontanylpalmitat, Dotriacontanylpalmitat, Tetratriacontanylpalmitat, Hexacosanylstearat, Octacosanylstearat, Triacontanylstearat, Dotriacontanylstearat, Tetratriacontanylstearat; Salicylaten, wie $C_1$-$C_{10}$-Salicylaten, z. B. Octylsalicylat; Benzoatestern, wie $C_{10}$-$C_{15}$-Alkylbenzoaten, Benzylbenzoat; anderen kosmetischen Estern, wie Fettsäuretriglyceriden, Propylenglykolmonolaurat, Polyethylenglykolmonolaurat, $C_{10}$-$C_{15}$-Alkyllactaten, etc. und Mischungen davon.

Geeignete Siliconöle B) sind z. B. lineare Polydimethylsiloxane, Poly(methylphenylsiloxane), cyclische Siloxane und Mischungen davon. Das zahlenmittlere Molekulargewicht der Polydimethylsiloxane und Poly(methylphenylsiloxane) liegt vorzugsweise in einem Bereich von etwa 1000 bis 150000 g/mol. Bevorzugte cyclische Siloxane weisen 4- bis 8-gliedrige Ringe auf. Geeignete cyclische Siloxane sind z. B. unter der Bezeichnung Cyclomethicon kommerziell erhältlich.

Bevorzugte Öl- bzw. Fettkomponenten B) sind ausgewählt unter Paraffin und Paraffinölen; Vaselin; natürlichen Fetten und Ölen, wie Castoröl, Sojaöl, Erdnussöl, Olivenöl, Sonnenblumenöl, Sesamöl, Avocadoöl, Kakaobutter, Mandelöl, Pfirsichkernöl, Ricinusöl, Lebertran, Schweineschmalz, Walrat, Spermacetöl, Spermöl, Weizenkeimöl, Macadamianussöl, Nachtkerzenöl, Jojobaöl; Fettalkoholen, wie Laurylalkohol, Myristylalkohol, Cetylalkohol, Stearylalkohol, Oleylalkohol, Cetylalkohol; Fettsäuren, wie Myristinsäure, Stearinsäure, Palmitinsäure, Ölsäure, Linolsäure, Linolensäure und davon verschiedenen gesättigten, ungesättigten und substituierten Fettsäuren; Wachsen, wie Bienenwachs, Carnaubawachs, Candilillawachs, Walrat sowie Mischungen der zuvor genannten Öl- bzw. Fettkomponenten.

Geeignete kosmetisch und pharmazeutisch verträgliche Öl- bzw. Fettkomponenten B) sind in Karl-Heinz Schrader, Grundlagen und Rezepturen der Kosmetika, 2. Auflage, Verlag Hüthig, Heidelberg, S. 319 - 355 beschrieben, worauf hier Bezug genommen wird.

Vorteilhafterweise werden diejenigen Öle, Fette und/oder Wachse gewählt, die auf Seite 28, Zeile 39 bis Seite 34, Zeile 22 der WO 2006/106140 beschrieben sind. Auf den Inhalt der genannten Textstelle wird hiermit in vollem Umfang Bezug genommen.

Der Gehalt an weiteren Ölen, Fetten und Wachsen beträgt höchstens 50, bevorzugt 30, weiter bevorzugt höchstens 20 Gewichts-%, bezogen auf das Gesamtgewicht der Zusammensetzung.

Geeignete hydrophile Träger B) sind ausgewählt unter Wasser, ein-, zwei- oder mehrwertigen Alkoholen mit vorzugsweise 1 bis 8 Kohlenstoffatomen, wie Ethanol, n-Propanol, iso-Propanol, Propylenglycol, Glycerin, Sorbit, etc.

Bei den erfindungsgemäßen kosmetischen Mitteln kann es sich um hautkosmetische, haarkosmetische, dermatologische, hygienische oder pharmazeutische Mittel handeln. Aufgrund ihrer filmbildenden Eigenschaften eignen sich die zuvor beschriebenen Copolymere und Polyelektrolytkomplexe insbesondere als Zusatzstoffe für Haar- und Hautkosmetika.

Vorzugsweise liegen die erfindungsgemäßen Mittel in Form eines Gels, Schaums, Sprays, einer Salbe, Creme, Emulsion, Suspension, Lotion, Milch oder Paste vor. Gewünschtenfalls können auch Liposomen oder Mikrosphären eingesetzt werden.

Die erfindungsgemäßen kosmetisch oder pharmazeutisch aktiven Mittel können zusätzlich kosmetisch und/oder dermatologisch aktive Wirkstoffe sowie Hilfsstoffe enthalten.

Vorzugsweise enthalten die erfindungsgemäßen kosmetischen Mittel wenigstens ein wie vorstehend definiertes Copolymer A), wenigstens einen wie vorstehend definierten Träger B) und wenigstens einen davon verschiedenen Bestandteil, der ausgewählt ist unter kosmetisch aktiven Wirkstoffen, Emulgatoren, Tensiden, Konservierungsmitteln, Parfümölen, Verdickern, Haarpolymeren, Haar- und Hautconditionern, Pfropfpolymeren, wasserlöslichen oder dispergierbaren silikonhaltigen Polymeren, Lichtschutzmitteln, Bleichmitteln, Gelbildnern, Pflegemitteln, Färbemitteln, Tönungsmitteln, Bräunungsmitteln, Farbstoffen, Pigmenten, Konsistenzgebern, Feuchthaltemitteln, Rückfettern, Collagen, Eiweisshydrolysaten, Lipiden, Antioxidantien, Entschäumern, Antistatika, Emollienzien und Weichmachern.

Die erfindungsgemäßen kosmetischen Zusammensetzungen können als wässrige oder wässrig-alkoholische

Lösungen, O/W- sowie W/O-Emulsionen, Hydrodispersionsformulierungen, feststoffstablilisierte Formulierungen, Stiftformulierungen, PIT-Formulierungen, in Form von Cremes, Schäumen, Sprays (Pumpspray oder Aerosol), Gelen, Gelsprays, Lotionen, Ölen, Ölgelen oder Mousse vorliegen und dementsprechend mit üblichen weiteren Hilfsstoffen formuliert werden.

Besonders bevorzugte kosmetische Zusammensetzungen im Sinne der vorliegenden Erfindung sind Shampoos und Haarpflegemittel. Die Erfindung betrifft demnach auch Zusammensetzungen zur Reinigung und/oder Pflege der Haare.

Insbesondere betrifft die Erfindung Haarpflegemittel ausgewählt aus der Gruppe bestehend aus Vorbehandlungsmitteln, Haarspülungen, Haarconditionern, Haarbalsamen, leave-on-Haarkuren, rinse-off-Haarkuren, Haarwässern, Pomaden, Frisiercremes, Frisierlotionen, Frisiergelen, Spitzenfluids, Hot-Oil-Treatments und Schaumkuren.

Weiterhin betrifft die Erfindung kosmetische Zusammensetzungen, die ausgewählt sind aus Gelcremes, Hydroformulierungen, Stiftformulierungen, kosmetischen Ölen und Ölgelen, Mascara, Selbstbräunern, Gesichtspflegemitteln, Körperpflegemitteln, After-Sun-Präparaten, Haarverformungsmitteln und Haarfestigern.

Weitere erfindungsgemäße kosmetische Zusammensetzungen sind hautkosmetische Zusammensetzungen, insbesondere solche zur Pflege der Haut. Diese liegen insbesondere als W/O- oder O/W-Hautcremes, Tag- und Nachtcremes, Augencremes, Gesichtscremes, Antifaltencremes, Mimikcremes, Feuchthaltecremes, Bleichcremes, Vitamincremes, Hautlotionen, Pflegelotionen und Feuchthaltelotionen vor.

Weiterhin eignen sich die erfindungsgemäßen Polymerkombinationen als Inhaltsstoffe für hautkosmetische Zubereitungen wie Gesichtswässer, Gesichtsmasken, Deodorantien und andere kosmetische Lotionen und für die Verwendung in der dekorativen Kosmetik, beispielsweise als Abdeckstift, Theaterfarbe, in Mascara und Lidschatten, Lippenstiften, Kajalstiften, Eyelinern, Makeup, Grundierungen, Rouges und Pudern und Augenbrauenstiften.

Außerdem können die erfindungsgemäßen Zusammensetzungen verwendet werden in Nose-Strips zur Porenreinigung, in Antiaknemitteln, Repellentien, Rasiermitteln, Haarentfernungsmitteln, Intimpflegemitteln, Fußpflegemitteln sowie in der Babypflege.

Weitere bevorzugte erfindungsgemäße Zusammensetzungen sind Wasch-, Dusch- und Badepräparate, die die erfindungsgemäßen Copolymere enthalten.

Unter Wasch-, Dusch- und Badepräparaten werden im Rahmen dieser Erfindung Seifen von flüssiger bis gelförmiger Konsistenz, wie Transparentseifen, Luxusseifen, Deoseifen, Cremeseifen, Babyseifen, Hautschutzseifen, Abrasivseifen und Syndets, pasteuse Seifen, Schmierseifen und Waschpasten, flüssige Wasch-, Dusch- und Badepräparate, wie Waschlotionen, Duschbäder und -gele, Schaumbäder, Ölbäder und Scrub-Präparate, Rasierschäume, -lotionen und - cremes verstanden.

Geeignete weitere Inhaltsstoffe für diese erfindungsgemäßen Wasch-, Dusch- und Badepräparate sind im Folgenden beschrieben.

Die Zusammensetzungen enthalten neben den erfindungsgemäßen Copolymeren weitere kosmetisch akzeptable Zusätze wie beispielsweise Emulgatoren und Co-Emulgatoren, Lösungsmittel, Tenside, Ölkörper, Konservierungsmittel, Parfümöle, kosmetische Pflege- und Wirkstoffe wie AHA-Säuren, Fruchtsäuren, Ceramide, Phytantriol, Collagen, Vitamine und Provitamine, beispielsweise Vitamin A, E und C, Retinol, Bisabolol, Panthenol, natürliche und synthetische Lichtschutzmittel, Naturstoffe, Trübungsmittel, Lösungsvermittler, Repellentien, Bleichmittel, Färbemittel, Tönungsmittel, Bräunungsmittel (z.B. Dihydroxyaceton), Mikropigmente wie Titanoxid oder Zinkoxid, Überfettungsmittel, Perlglanzwachse, Konsistenzgeber, Verdicker, Solubilisatoren, Komplexbildner, Fette, Wachse, Silikonverbindungen, Hydrotrope, Farbstoffe, Stabilisatoren, pH-Wert Regulatoren, Reflektoren, Proteine und Proteinhydrolysate (z.B. Weizen-, Mandel- oder Erbsenproteine), Ceramid, Eiweißhydrolysate, Salze, Gelbildner, Konsistenzgeber, Silikone, Feuchthaltemittel (z.B. 1,2-Pentandiol), Rückfetter, UV-Lichtschutzfilter und weitere übliche Additive. Des weiteren können zur Einstellung der jeweils gewünschten Eigenschaften insbesondere auch weitere Polymere enthalten sein.

Die erfindungsgemäßen kosmetischen Zusammensetzungen enthalten die erfindungsgemäßen Copolymere in einer Menge von 0,01 bis 10 Gew.-%, bevorzugt 0,05 bis 5 Gew.-%, besonders bevorzugt 0,1 bis 1,5 Gew.-%, bezogen auf das Gewicht der Zusammensetzung.

In einer bevorzugten Ausführungsform der Erfindung enthalten die erfindungsgemäßen Wasch-, Dusch- und Badepräparate sowie Shampoos und Haarpflegemittel weiterhin wenigstens ein Tensid.

In einer weiteren bevorzugten Ausführungsform der Erfindung enthalten die erfindungsgemäßen Shampoos und Haarpflegemittel neben den Polymeren weiterhin wenigstens eine Öl- und/oder Fettphase und ein Tensid.

Tenside

Als Tenside können anionische, kationische, nichtionische und/oder amphotere Tenside eingesetzt werden.

Vorteilhafte waschaktive anionische Tenside im Sinne der vorliegenden Erfindung sind

- Acylaminosäuren und deren Salze, wie Acylglutamate, insbesondere Natriumacylglutamat

- Sarcosinate, beispielsweise Myristoyl Sarcosin, TEA-Lauroyl Sarcosinat, Natriumlauroylsarcosinat und Natriumcocoylsarcosinat,

  Sulfonsäuren und deren Salze, wie
- Acylisethionate, beispielsweise Natrium- oder Ammoniumcocoylisethionat
- Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat, Dinatrium PEG-5 Laurylcitratsulfosuccinat und Derivate,
- Alkylethersulfate, beispielsweise Natrium-, Ammonium-, Magnesium-, MIPA-, TIPA-Laurethsulfat, Natriummyrethsulfat und Natrium $C_{12-13}$ Parethsulfat,
- Alkyethersulfonate, beispielsweise Natrium C12-15 Pareth-15 Sulfonat
- Alkylsulfate, beispielsweise Natrium-, Ammonium- und TEA-Laurylsulfat.

  Weitere vorteilhafte anionische Tenside sind
- Taurate, beispielsweise Natriumlauroyltaurat und Natriummethylcocoyltaurat,
- Ether-Carbonsäuren, beispielsweise Natriumlaureth-13 Carboxylat und Natrium PEG-6 Cocamide Carboxylat, Natrium PEG-7-Olivenöl-Carboxylat
- Phosphorsäureester und Salze, wie beispielsweise DEA-Oleth-10 Phosphat und Dilaureth-4 Phosphat,
- Alkylsulfonate, beispielsweise Natriumcocosmonoglyceridsulfat, Natrium $C_{12-14}$ Olefinsulfonat, Natriumlaurylsulfoacetat und Magnesium PEG-3 Cocamidsulfat,
- Acylglutamate wie Di-TEA-palmitoylaspartat und Natrium Caprylic/Capric Glutamat,
- Acylpeptide, beispielsweise Palmitoyl hydrolysiertes Milchprotein, Natrium Cocoyl hydrolysiertes Sojaprotein und Natrium-/Kalium Cocoyl hydrolysiertes Kollagen sowie Carbonsäuren und Derivate, wie beispielsweise Laurinsäure, Aluminiumstearat, Magnesiumalkanolat und Zinkundecylenat, Ester-Carbonsäuren, beispielsweise Calciumstearoyllactylat, Laureth-6 Citrat und Natrium PEG-4 Lauramidcarboxylat
- Alkylarylsulfonate.

Vorteilhafte waschaktive kationische Tenside im Sinne der vorliegenden Erfindung sind quaternäre Tenside. Quaternäre Tenside enthalten mindestens ein N-Atom, das mit 4 Alkyl- oder Arylgruppen kovalent verbunden ist. Vorteilhaft sind beispielsweise Alkylbetain, Alkylamidopropylbetain und Alkylamidopropylhydroxysultain.

Weitere vorteilhafte kationische Tenside im Sinne der vorliegenden Erfindung sind ferner

- Alkylamine,
- Alkylimidazole und
- ethoxylierte Amine

und insbesondere deren Salze.

Vorteilhafte waschaktive amphotere Tenside im Sinne der vorliegenden Erfindung sind Acyl-/dialkylethylendiamine, beispielsweise Natriumacylamphoacetat, Dinatriumacylamphodipropionat, Dinatriumalkylamphodiacetat, Natriumacylamphohydroxypropylsulfonat, Dinatriumacylamphodiacetat, Natriumacylamphopropionat, und N-Kokosfettsäureamidoethyl-N-hydroxyethylglycinat Natriumsalze.

Weitere vorteilhafte amphotere Tenside sind N-Alkylaminosäuren, beispielsweise Aminopropylalkylglutamid, Alkylaminopropionsäure, Natriumalkylimidodipropionat und Lauroamphocarboxyglycinat.

Vorteilhafte waschaktive nicht-ionische Tenside im Sinne der vorliegenden Erfindung sind

- Alkanolamide, wie Cocamide MEA/DEA/MIPA,
- Ester, die durch Veresterung von Carbonsäuren mit Ethylenoxid, Glycerin, Sorbitan oder anderen Alkoholen entstehen,
- Ether, beispielsweise ethoxylierte Alkohole, ethoxyliertes Lanolin, ethoxylierte Polysiloxane, propoxylierte POE Ether, Alkylpolyglycoside wie Laurylglucosid, Decylglycosid und Cocoglycosid, Glycoside mit einem HLB-Wert von wenigstens 20 (z.B. Belsil®SPG 128V (Wacker)).

Weitere vorteilhafte nicht-ionische Tenside sind Alkohole und Aminoxide, wie Cocoamidopropylamin-Oxid.

Bevorzugte anionische, amphotere und nichtionische Shampoo-Tenside sind beispielsweise in "Kosmetik und Hygiene von Kopf bis Fuß", Hrsg. W. Umbach, 3. Auflage, Wiley-VCH, 2004, S.131-134 genannt, worauf an dieser Stelle in vollem Umfang Bezug genommen wird.

Unter den Alkylethersulfaten sind insbesondere Natriumalkylethersulfate auf Basis von zwei- oder dreifach ethoxyliertem Lauryl- und Myristylalkohol bevorzugt. Sie übertreffen die Alkylsulfate deutlich bezüglich der Unempfindlichkeit gegen Wasserhärte, der Verdickbarkeit, der Kältelöslichkeit und insbesondere der Haut- und Schleimhautverträglichkeit. Sie können auch als alleinige Waschrohstoffe für Shampoos eingesetzt werden. Laurylethersulfat weist

bessere Schaumeigenschaften als Myristylethersulfat auf, ist diesem aber in der Milde unterlegen.

Alkylethercarboxylate gehören zu den mildesten Tensiden überhaupt, zeigen aber ein schlechtes Schaum- und Viskositätsverhalten. Sie werden oft in Kombination mit Alkylethersulfaten und amphoteren Tensiden in Haarwaschmitteln eingesetzt. Sulfobernsteinsäureester (Sulfosuccinate) sind mild und gut schäumende Tenside werden aber wegen ihrer schlechten Verdickbarkeit bevorzugt nur zusammen mit anderen anionischen und amphoteren Tensiden und wegen ihrer geringen Hydrolysestabilität bevorzugt nur in neutralen bzw. gut gepufferten Produkten eingesetzt. Amidopropylbetaine sind als alleinige Waschrohstoffe praktisch unbedeutend, da ihr Schaumverhalten sowie ihre Verdickbarkeit nur mäßig ausgeprägt sind. Demgegenüber weisen diese Tenside eine ausgezeichnete Haut- und Augenschleimhautverträglichkeit auf. In Kombination mit anionischen Tensiden lässt sich deren Milde synergistisch verbessern. Bevorzugt ist die Verwendung von Cocamidopropylbetain. Amphoacetate /Amphodiacetate besitzen als amphotere Tenside eine sehr gute Haut- und Schleimhautverträglichkeit und können haarkonditionierend wirken bzw. die Pflegewirkung von Zusatzstoffen erhöhen. Sie werden ähnlich wie die Betaine zur Optimierung von Alkylethersulfat-Formulierungen eingesetzt. Am meisten bevorzugt sind Natriumcocoamphoacetat und Dinatriumcocoamphodiacetat.

Alkylpolyglykoside sind nichtionische Waschrohstoffe. Sie sind mild, haben gute Universaleigenschaften, schäumen jedoch schwach. Aus diesem Grund werden sie bevorzugt in Kombinationen mit anionischen Tensiden verwendet.

Sorbitanester gehören ebenfalls zu den nichtionischen Waschrohstoffen. Wegen ihrer ausgezeichneten Milde werden sie bevorzugt zum Einsatz bei Baby-Shampoos verwendet. Als Schwachschäumer werden sie bevorzugt in Kombination mit anionischen Tensiden eingesetzt.

Es ist vorteilhaft, das oder die waschaktiven Tenside aus der Gruppe der Tenside zu wählen, welche einen HLB-Wert von mehr als 25 haben, besonders vorteilhaft sind solche, welchen einen HLB-Wert von mehr als 35 haben.

Es ist erfindungsgemäß von Vorteil, wenn ein oder mehrere dieser Tenside in einer Konzentration von 1 bis 30 Gewichts-%, bevorzugt in einer Konzentration von 5 bis 25 Gewichts-% und ganz besonders bevorzugt in einer Konzentration von 10 bis 20 Gewichts-%, jeweils bezogen auf das Gesamtgewicht der Zusammensetzung eingesetzt werden.

Polysorbate

Als waschaktive Agentien können auch Polysorbate vorteilhaft in die erfindungsgemäßen Zusammensetzungen eingearbeitet werden.

Im Sinne der Erfindung vorteilhafte Polysorbate sind beispielsweise

- Polyoxyethylen(20)sorbitanmonolaurat (Tween®20, CAS-Nr. 9005-64-5)
- Polyoxyethylen(4)sorbitanmonolaurat (Tween®21, CAS-Nr. 9005-64-5)
- Polyoxyethylen(4)sorbitanmonostearat (Tween®61, CAS-Nr. 9005-67-8)
- Polyoxyethylen(20)sorbitantristearat (Tween®65, CAS-Nr. 9005-71 -4)
- Polyoxyethylen(20)sorbitanmonooleat (Tween®80, CAS-Nr. 9005-65-6)
- Polyoxyethylen(5)sorbitanmonooleat (Tween®81, CAS-Nr. 9005-65-5)
- Polyoxyethylen(20)sorbitantrioleat (Tween®85, CAS-Nr. 9005-70-3).

  Besonders vorteilhaft sind
- Polyoxyethylen(20)sorbitanmonopalmitat (Tween®40, CAS-Nr. 9005-66-7) und
- Polyoxyethylen(20)sorbitanmonostearat (Tween®60, CAS-Nr. 9005-67-8).

Die Polysorbate werden vorteilhaft in einer Konzentration von 0,1 bis 5 und insbesondere in einer Konzentration von 1,5 bis 2,5 Gew.-%, bezogen auf das Gesamtgewicht der Zusammensetzung einzeln oder als Mischung mehrer Polysorbate, eingesetzt.

Weitere Konditionierungsmittel

Sofern gewünscht, werden zusätzlich zu den erfindungsgemäßen Copolymeren als weitere Konditionierungsmittel für die erfindungsgemäßen kosmetischen Zusammensetzungen bevorzugt diejenigen Konditionierungsmittel gewählt, die auf Seite 34, Zeile 24 bis Seite 37, Zeile 10 der WO 2006/106140 beschrieben sind. Auf den Inhalt der genannten Textstelle wird hiermit in vollem Umfang Bezug genommen.

Rheologiemodifizierungsmittel

Bei den geeigneten Rheologiemodifizierungsmitteln handelt es sich vor allem um Verdicker. Für Shampoos und Haarpflegemittel geeignete Verdicker sind in "Kosmetik und Hygiene von Kopf bis Fuß", Hrsg. W. Umbach, 3.

Auflage, Wiley-VCH, 2004, S.235-236 genannt, worauf an dieser Stelle vollumfänglich Bezug genommen wird. Geeignete Verdickungsmittel für die erfindungsgemäßen kosmetischen Zusammensetzungen sind beispielsweise auch auf Seite 37, Zeile 12 bis Seite 38, Zeile 8 der WO 2006/106140 beschrieben. Auf den Inhalt der genannten Textstelle wird hiermit in vollem Umfang Bezug genommen.

Konservierungsmittel

Die erfindungsgemäßen kosmetischen Zusammensetzungen können auch Konservierungsmittel enthalten. Zusammensetzungen mit hohen Wassergehalten müssen zuverlässig vor Verkeimung geschützt sein. Geeignete Konservierungsmittel für die erfindungsgemäßen kosmetischen Zusammensetzungen sind beispielsweise auf Seite 38, Zeile 10 bis Seite 39, Zeile 18 der WO 2006/106140 beschrieben. Auf den Inhalt der genannten Textstelle wird hiermit in vollem Umfang Bezug genommen.

Komplexbildner: Da die Rohstoffe und auch die Shampoos selbst überwiegend in Stahlapparaturen hergestellt werden, können die Endprodukte Eisen(-Ionen) in Spurenmengen enthalten. Um zu verhindern, dass diese Verunreinigungen über Reaktionen mit Farbstoffen und Parfümölbestandteilen die Produktqualität nachteilig beeinflussen, werden Komplexbildner wie Salze der Ethylendiamintetraessigsäure, der Nitrilotriessigsäure, der Iminodibernsteinsäure oder Phosphate zugesetzt.

UV- Lichtschutzfilter: Um die in den erfindungsgemäßen Zusammensetzungen enthaltenen Inhaltsstoffe wie beispielsweise Farbstoffe und Parfümöle gegen Veränderungen durch UV-Licht zu stabilisieren, können UV- Lichtschutzfilter, wie z. B. Benzophenon-Derivate, eingearbeitet werden. Geeignete UV- Lichtschutzfilter für die erfindungsgemäßen kosmetischen Zusammensetzungen sind beispielsweise auf Seite 39, Zeile 20 bis Seite 41 Zeile 10 der WO 2006/106140 beschrieben. Auf den Inhalt der genannten Textstelle wird hiermit in vollem Umfang Bezug genommen.

Antioxidantien: Ein Gehalt der erfindungsgemäßen Zusammensetzungen an Antioxidantien ist im allgemeinen bevorzugt. Erfindungsgemäß können als Antioxidantien alle für kosmetische Anwendungen geeigneten oder gebräuchlichen Antioxidantien verwendet werden. Geeignete Antioxidantien für die erfindungsgemäßen kosmetischen Zusammensetzungen sind beispielsweise auf Seite 41, Zeile 12 bis Seite 42 Zeile 33 der WO 2006/106140 beschrieben. Auf den Inhalt der genannten Textstelle wird hiermit in vollem Umfang Bezug genommen.

Puffer: Puffer gewährleisten die pH-Wert-Stabilität der Zusammensetzungen. Überwiegend verwendet werden Citrat-, Lactat- und Phosphat-Puffer.

Lösungsvermittler: Sie werden eingesetzt, um pflegende Öle oder Parfümöle klar in Lösung zu bringen und auch in der Kälte klar in Lösung zu halten. Die gängigsten Lösungsvermittler sind ethoxylierte nichtionische Tenside, z. B. hydrierte und ethoxylierte Ricinusöle.

Keimhemmende Mittel: Weiterhin können auch keimhemmende Mittel eingesetzt werden. Dazu gehören generell alle geeigneten Konservierungsmittel mit spezifischer Wirkung gegen grampositive Bakterien, z.B. Triclosan (2,4,4'-Trichlor-2'-hydroxydiphenylether), Chlorhexidin (1,1'-Hexamethylenbis[5-(4-chlorphenyl)-biguanid) sowie TTC (3,4,4'-Trichlorcarbanilid). Quartäre Ammonium-Verbindungen sind prinzipiell ebenfalls geeignet und werden bevorzugt für desinfizierende Seifen und Waschlotionen verwendet. Auch zahlreiche Riechstoffe haben antimikrobielle Eigenschaften. Auch eine große Anzahl etherischer Öle bzw. deren charakteristische Inhaltsstoffe wie z.B. Nelkenöl (Eugenol), Minzöl (Menthol) oder Thymianöl (Thymol), zeigen eine ausgeprägte antimikrobielle Wirksamkeit.

Die antibakteriell wirksamen Stoffe werden in der Regel in Konzentrationen von ca. 0,1 bis 0,3 Gew.-% eingesetzt.

Dispergiermittel: Wenn in den erfindungsgemäßen Zusammensetzungen unlösliche Wirkstoffe, z.B. Antischuppenwirkstoffe oder Siliconöle, dispergiert und auf Dauer in Schwebe gehalten werden sollen, müssen Dispergiermittel und Verdicker wie z. B. Magnesium-Aluminium-Silicate, Bentonite, Fettacyl-Derivate, Polyvinylpyrrolidon oder Hydrokolloide, z. B. Xanthan Gum oder Carbomere, eingesetzt werden. Erfindungsgemäß sind Konservierungsmittel in einer Gesamtkonzentration von höchstens 2, bevorzugt höchstens 1,5 und besonders bevorzugt höchstens 1 Gewichts-%, bezogen auf das Gesamtgewicht der Zusammensetzung, enthalten.

Die Zusammensetzungen können außer den vorgenannten Substanzen gegebenenfalls die in der Kosmetik üblichen Zusatzstoffe, beispielsweise Parfüm, Farbstoffe, rückfettende Agentien, Komplexierungs- und Sequestrierungsagentien, Perlglanzagentien, Pflanzenextrakte, Vitamine, Wirkstoffe, Pigmente, die eine färbende Wirkung haben, weichmachende, anfeuchtende und/oder feuchthaltende Substanzen, oder andere übliche Bestandteile einer kosmetischen oder dermatologischen Formulierung wie Alkohole, Polyole, Polymere, organische Säuren zur pH-Wert-Einstellung, Schaumstabilisatoren, Elektrolyte, organische Lösemittel oder Silikonderivate enthalten.

Hinsichtlich der genannten, dem Fachmann bekannten weiteren Inhaltsstoffe für die Zusammensetzungen sei auf "Kosmetik und Hygiene von Kopf bis Fuß", Hrsg. W. Umbach, 3. Auflage, Wiley-VCH, 2004, S.123-128 verwiesen, worauf an dieser Stelle vollumfänglich Bezug genommen wird.

Die erfindungsgemäßen Polymere sind geeignet, die Abscheidungsmenge und - geschwindigkeit sowie die Verweilzeit von weiteren Wirkstoffen, wie beispielsweise Silikonen oder UV-Lichtschutzfiltern, auf der Haut und/oder

dem Haar zu verstärken bzw. zu erhöhen. Substanzen oder Mittel, die solche Effekte besitzen, werden auch als Depositioning Aids bezeichnet.

US 6,998,113 beschreibt rinse-off-Zubereitungen, die dazu führen, dass die damit behandelte Haut effektiv vor UV-Strahlung geschützt wird. Einige der dort beschriebenen Zubereitungen enthalten kationische Polymere. Im Sinne der vorliegenden Erfindung können auch die erfindungsgemäßen Copolymere in den Zubereitungen der US 6,998,113 verwendet werden. Insbesondere können die erfindungsgemäßen Copolymere für den von der US 6,998,113 genannten Zweck in Sonnenschutz-Wasch- und Badepräparaten eingesetzt werden. Auf die Offenbarung der US 6,998,113 wird hiermit in vollem Umfang Bezug genommen.

Geeignete Silikone sind beispielsweise in der US 5,935,561, Spalte 13, Z.64 bis Spalte 18, Z.61 dargestellt, worauf hiermit in vollem Umfang Bezug genommen wird.

Stellvertretend seien genannt:

- Dimethicone
- Polyalkyl -oder Polyarylsiloxane (US 5,935,561, Spalte 13, Formel I)
- Alkylamino substituierte Silikone (US 5,935,561, Spalte 14, Formel II (Amodimethicone))
- Kationische Silikone (US 5,935,561, Spalten 14 und 15, Formel III)
- Trimethylsilylamodimethicone (US 5,935,561, Spalte 15, Formel IV)
- Silikone gemäß US 5,935,561, Spalte 15, Formel V
- cyclische Polysiloxane gemäß US 5,935,561, Spalte 16, Formel VI
- Ethoxylierte Glycerin-Fettsäureester

Die erfindungsgemäßen Zusammensetzungen wie Shampoos und Haarpflegemittel enthalten gegebenenfalls ethoxylierte Öle ausgewählt aus der Gruppe der ethoxylierten Glycerin-Fettsäureester, insbesondere bevorzugt PEG-10 Olivenölglyceride, PEG-11 Avocadoölglyceride, PEG-11 Kakaobutterglyceride, PEG-13 Sonnenblumenölglyceride, PEG-15 Glycerylisostearat, PEG-9 Kokosfettsäureglyceride, PEG-54 Hydriertes Ricinusöl, PEG-7 Hydriertes Ricinusöl, PEG-60 Hydriertes Ricinusöl, Jojobaöl Ethoxylat (PEG-26 Jojoba-Fett-Säuren, PEG-26 Jojobaalkohol), Glycereth-5 Cocoat, PEG-9 Kokosfettsäureglyceride, PEG-7 Glycerylcocoat, PEG-45 Palmkernölglyceride, PEG-35 Ricinusöl, Olivenöl-PEG-7 Ester, PEG-6 Caprylisäure/Caprinsäureglyceride, PEG-10 Olivenölglyceride, PEG-13 Sonnenblumenölglyceride, PEG-7 Hydriertes Ricinusöl, Hydrierte Palmkernölglycerid-PEG-6 Ester, PEG-20 Maisölglyceride, PEG- 18 Glyceryloleat-cocoat, PEG-40 Hydriertes Ricinusöl, PEG-40 Ricinusöl, PEG-60 Hydriertes Ricinusöl, PEG-60 Maisölglyceride, PEG-54 Hydriertes Ricinusöl, PEG-45 Palmkernölglyceride, PEG-80 Glycerylcocoat, PEG-60 Mandelölglyceride, PEG-60 "Evening Primrose" Glyceride, PEG-200 Hydriertes Glycerylpalmat, PEG-90 Glycerylisostearat. Bevorzugte ethoxylierte Öle sind PEG-7 Glycerylcocoat, PEG-9 Kokosglyceride, PEG-40 Hydriertes Rizinusöl, PEG-200 hydriertes Glycerylpalmat.

Ethoxylierte Glycerin-Fettsäureester werden in wässrigen Reinigungsrezepturen zu verschiedenen Zwecken eingesetzt. Glycerin-Fettsäureester mit einem Ethoxylierungsgrad von ca. 30-50 dienen als Lösungsvermittler für unpolare Substanzen wie Parfumöle. Hochethoxylierte Glycerin-Fettsäureester werden als Verdicker eingesetzt.

Wirkstoffe

In die erfindungsgemäßen Zusammensetzungen lassen sich verschiedenste Wirkstoffe mit unterschiedlicher Löslichkeit homogen einarbeiten. Vorteilhafte Wirkstoffe in den erfindungsgemäßen kosmetischen Zusammensetzungen sind beispielsweise auf Seite 44, Zeile 24 bis Seite 49 Zeile 39 der WO 2006/106140 beschrieben. Auf den Inhalt der genannten Textstelle wird hiermit in vollem Umfang Bezug genommen.

UV-Lichtschutzmittel

Die erfindungsgemäßen Zusammensetzungen enthalten in einer bevorzugten Ausführungsform UV-Lichtschutzmittel zum Schutz der Haut und/oder der Haare. Geeignete UV-Lichtschutzmittel sind in der WO 2006/106114, S.24, Z.4 bis S.27, Z.27 ausführlich beschrieben, worauf hiermit in vollem Umfang Bezug genommen wird.

Vorteilhaft enthalten die Zusammensetzungen Substanzen, die UV-Strahlung im UVB-Bereich und Substanzen, die UV-Strahlung im UVA-Bereich absorbieren, wobei die Gesamtmenge der Filtersubstanzen z.B. 0,1 bis 30 Gew.-%, vorzugsweise 0,5 bis 20 Gew.-%, insbesondere 1 bis 15 Gew.-% beträgt, bezogen auf das Gesamtgewicht der Zusammensetzungen, um kosmetische Zusammensetzungen zur Verfügung zu stellen, welche die Haut vor dem gesamten Bereich der ultravioletten Strahlung schützen.

Der größte Teil der Lichtschutzmittel in den zum Schutz der menschlichen Epidermis dienenden kosmetischen oder dermatologischen Zusammensetzungen besteht aus Verbindungen, die UV-Licht im UV-B-Bereich absorbieren. Beispielsweise beträgt der Anteil der erfindungsgemäß zu verwendenden UV-A-Absorber 10 bis 90 Gew.-%, bevorzugt

20 bis 50 Gew.-% bezogen auf die Gesamtmenge von UV-B und UV-A absorbierenden Substanzen.

Perlglanzwachse

Geeignete Perlglanzwachse für die erfindungsgemäßen kosmetischen Zusammensetzungen sind beispielsweise auf Seite 50, Zeile 1 bis Zeile 16 der WO 2006/106140 beschrieben. Auf den Inhalt der genannten Textstelle wird hiermit in vollem Umfang Bezug genommen.

Die erfindungsgemäßen Zusammensetzungen können weiterhin Glitterstoffe und/oder andere Effektstoffe (z.B. Farbschlieren) enthalten.

Emulgatoren

Die erfindungsgemäßen kosmetischen Zusammensetzungen liegen in einer bevorzugten Ausführungsform der Erfindung in Form von Emulsionen vor. Die Herstellung solcher Emulsionen erfolgt nach bekannten Methoden. Geeignete Emulgatoren für die erfindungsgemäßen Emulsionen sind beispielsweise auf Seite 50, Zeile 18 bis Seite 53, Zeile 4 der WO 2006/106140 beschrieben. Auf den Inhalt der genannten Textstelle wird hiermit in vollem Umfang Bezug genommen.

Parfümöle

Sollen den erfindungsgemäßen kosmetischen Zusammensetzungen Parfumöle zugesetzt werden, so sind geeignete Parfümöle beispielsweise auf Seite 53, Zeile 10 bis Seite 54, Zeile 3 der WO 2006/106140 beschrieben. Auf den Inhalt der genannten Textstelle wird hiermit in vollem Umfang Bezug genommen.

Pigmente

Gegebenenfalls enthalten die erfindungsgemäßen kosmetischen Zusammensetzungen weiterhin Pigmente. Die Pigmente liegen im Produkt meist in ungelöster Form vor und können in einer Menge von 0,01 bis 25 Gew.%, besonders bevorzugt von 5 bis 15 Gew.% enthalten sein. Die bevorzugte Teilchengröße beträgt 1 bis 200 μm, insbesondere 3 bis 150 μm, besonders bevorzugt 10 bis 100 μm.

Geeignete Pigmente für die erfindungsgemäßen Zusammensetzungen sind beispielsweise auf Seite 54, Zeile 5 bis Seite 55, Zeile 19 der WO 2006/106140 beschrieben. Auf den Inhalt der genannten Textstelle wird hiermit in vollem Umfang Bezug genommen.

Polymere

Die erfindungsgemäßen kosmetischen Zusammensetzungen enthalten in einer bevorzugten Ausführungsform außer dem nach dem erfindungsgemäßen Verfahren hergestellten Polymer weitere Polymere.

Geeignete zusätzliche Polymere für die erfindungsgemäßen Zusammensetzungen sind beispielsweise auf Seite 55, Zeile 21 bis Seite 63, Zeile 2 der WO 2006/106140 beschrieben. Auf den Inhalt der genannten Textstelle wird hiermit in vollem Umfang Bezug genommen.

Shampoo-Typen

Eine bevorzugte Ausführungsform der Erfindung sind Haar-Shampoos enthaltend die erfindungsgemäßen Copolymere. An Shampoos werden je nach Haarqualität oder Kopfhautproblem gegebenenfalls zusätzliche Anforderungen gestellt. Die Wirkungsweise der bevorzugten Shampoo-Typen mit den wichtigsten zusätzlichen Wirkungen bzw. wichtigsten speziellen Zielsetzungen wird nachfolgend beschrieben. Erfindungsgemäß bevorzugt sind beispielsweise Shampoos für normales bzw. schnell fettendes bzw. geschädigtes Haar, Antischuppenshampoos, Babyshampoos und 2-in-1-Shampoos (also Shampoo und Spülung in einem).

Erfindungsgemäße Shampoos für normales Haar: die Haarwäsche soll Haar und Kopfhaut von dem in Talgdrüsen gebildeten Hautfett, den aus Schweissdrüsen mit Wasser austretenden anorganischen Salzen, Aminosäuren, Harnstoff und Milchsäure, abgeschilferten Hautpartikeln, Umweltschmutz, Gerüchen und gegebenenfalls Rückständen haarkosmetischer Behandlungen befreien. Normales Haar bedeutet kurzes bis schulterlanges Haar, welches nur schwach geschädigt ist. Dementsprechend soll der Anteil an Konditionierhilfsstoffen auf diesen Haartyp optimiert sein.

Erfindungsgemäße Shampoos für schnell fettendes Haar: eine erhöhte Fettproduktion der Talgdrüsen der Kopfhaut führt bereits 1-2 Tage nach der Haarwäsche zu einer strähnigen, unansehnlichen Frisur. Öl- und wachsartige Hautfettbestandteile beschweren das Haar und mindern die Reibung von Haar zu Haar und verringern damit den Frisurenhalt. Das eigentliche haarkosmetische Problem bei schnell fettenden Haaren ist also das vorzeitige Zusammenfallen voluminöser Frisuren. Um dies zu vermeiden, muss man verhindern, dass die Haaroberfläche beschwert und zu glatt und geschmeidig wird. Dies wird bevorzugt durch die Tensidgrundlage aus gut reinigenden und besonders wenig substantiv ausgeprägten Waschrohstoffen erreicht. Zusätzliche Pflegestoffe, die sich zum Hautfett addieren würden, wie rückfettende Substanzen werden in Shampoos für schnell fettendes Haar nicht oder nur mit größter Vorsicht eingesetzt. Erfindungsgemäße volumengebende Shampoos für feines Haar können vergleichbar formuliert werden.

Erfindungsgemäße Shampoos für trockenes, strapaziertes (geschädigtes) Haar: Die Struktur des Haares wird im Laufe des Haarwachstums durch mechanische Einflüsse wie Kämmen, Bürsten und vor allen Dingen Toupieren (Kämmen gegen die Wuchsrichtung), durch die Einwirkung von UV-Strahlung bzw. sichtbarem Licht und durch kosmetische Behandlungen, wie Dauerwellen, Blondierung oder Färbung verändert. Die Schuppenschicht der Haare weist von der Wurzel bis zur Spitze ein zunehmend strapazierteres Aussehen auf; in Extremfällen ist sie an der Spitze völlig abgetragen, und die Haarspitzen sind gespalten (Haarspliss). Geschädigtes Haar kann grundsätzlich nicht mehr in den Zustand des gesunden Haarnachwuchses zurückgeführt werden. Es gelingt aber, diesem Idealzustand hinsichtlich Griff, Glanz und Kämmbarkeit durch Verwendung von erfindungsgemäßen Shampoos mit gegebenenfalls hohen Anteilen an pflegenden Stoffen (Konditioniermitteln) sehr nahe zu kommen.

Eine noch bessere haarkonditionierende Wirkung als mit einem Shampoo wird mit einem erfindungsgemäßen Haarpflegemittel beispielsweise in Form einer Spülungs- oder Kurmittelbehandlung nach der Haarwäsche erreicht. Spülungs- oder Kurmittel für Haare, die erfindungsgemäße Copolymere enthalten, sind ebenfalls erfindungsgemäß. Erfindungsgemäße 2-in-1- Shampoos sind besonders stark pflegende Shampoos, bei denen durch die Konzipierung als "Shampoo und Spülung in einem" der Zusatznutzen Pflege gleichwertig neben den Grundnutzen Reinigung gestellt wird. Erfindungsgemäße 2-in-1-Zusammensetzungen enthalten erhöhte Mengen an Konditioniermitteln. Antischuppenshampoos: Erfindungsgemäße Antischuppenshampoos haben im Vergleich zu Antischuppenhaarwässern den Vorteil, dass sie nicht nur durch entsprechende Wirkstoffe gegen Schuppenbefall die Bildung neuer sichtbarer Schuppen verringern und bei langfristiger Anwendung verhindern, sondern mit der Haarwäsche auch bereits abgeschilferte Schuppen entfernen. Nach dem Ausspülen der Waschflotte bleibt allerdings nur ein geringer, jedoch ausreichender Anteil der Wirkstoffe auf Kopfhaut und Haar zurück. Es gibt verschiedene Antischuppen-Wirkstoffe, die in die erfindungsgemäßen Shampoo-Zusammensetzungen eingearbeitet werden können, wie z. B. Zinkpyrithion, Ketokonazol, Elubiol, Clotrimazol, Climbazol oder Piroctón Olamin. Zusätzlich weisen diese Substanzen eine die Abschilferung normalisierende Wirkung auf.

Die Grundlage von Antischuppenshampoos entspricht überwiegend der Formulierung von Shampoos für normales Haar mit gutem Reinigungseffekt.

Babyshampoos: bei einer bevorzugten Ausführungsform der Erfindung handelt es sich bei den erfindungsgemäßen Shampoo-Zubereitungen um Babyshampoos. Diese sind optimal haut- und schleimhautverträglich. Kombinationen von Waschrohstoffen mit sehr guter Hautverträglichkeit bilden die Basis dieser Shampoos. Zusätzliche Stoffe zur weiteren Verbesserung der Haut- und Schleimhautverträglichkeit und der Pflegeeigenschaften werden vorteilhaft zugefügt, wie z. B. nichtionische Tenside, Proteinhydrolysate und Panthenol oder Bisabolol. Alle notwendigen Rohstoffe und Hilfsstoffe, wie Konservierungsmittel, Parfümöle, Farbstoffe usw., werden unter dem Aspekt einer hohen Verträglichkeit und Milde ausgewählt.

Shampoos für trockene Kopfhaut: bei einer weiteren bevorzugten Ausführungsform der Erfindung handelt es sich bei den erfindungsgemäßen Shampoo-Zubereitungen um Shampoos für trockene Kopfhaut. Das primäre Ziel dieser Shampoos ist die Verhinderung der Austrocknung der Kopfhaut, da trockene Kopfhaut zu Juckreiz, Rötung und Entzündung führen kann. Wie auch bei den Babyshampoos bilden Kombinationen von Waschrohstoffen mit sehr guter Hautverträglichkeit die Basis dieser Shampoos. Zusätzlich können gegebenenfalls Rückfetter und Feuchthaltemittel, wie z. B. Glycerin oder Harnstoff, eingesetzt werden.

Die erfindungsgemäßen Shampoo-Zusammensetzungen können auch als Shampookonzentrate mit erhöhten Tensidgehalten von 20-30 Gew.-% vorliegen. Sie basieren auf speziellen Waschrohstoffkombinationen und Konsistenzregulatoren, die die gute Verteilbarkeit und das spontane Anschäumvermögen auch einer kleinen Anwendungsmenge gewährleisten. Ein besonderer Vorteil ist beispielsweise die Möglichkeit, die Ergiebigkeit von 200 ml Shampoo mit einer 100-ml-Flasche zu erreichen.

Darreichung

Es ist vorteilhaft, wenn die erfindungsgemäßen Zusammensetzungen in einer Flasche oder Quetschflasche aufbewahrt und aus dieser heraus angewendet werden. Demzufolge sind auch Flaschen oder Quetschflaschen, welche eine erfindungsgemäße Zusammensetzung enthalten, erfindungsgemäß.

Die erfindungsgemäßen Copolymere, wie zuvor definiert, können bevorzugt in Shampooformulierungen insbesondere als Konditioniermittel eingesetzt werden. Bevorzugte Shampooformulierungen enthalten

a) 0,05 bis 10 Gew.-% wenigstens eines erfindungsgemäßen Copolymers,

b) 25 bis 94,95 Gew.-% Wasser,
c) 5 bis 50 Gew.-% Tenside,
c) 0 bis 5 Gew.-% eines weiteren Konditioniermittels,
d) 0 bis 10 Gew.-% weitere kosmetische Bestandteile.

In den Shampooformulierungen können alle in Shampoos üblicherweise eingesetzten anionischen, neutralen, amphoteren oder kationischen Tenside verwendet werden. Geeignete Tenside wurden vorstehend genannt.

Seifen und Syndets

Weitere erfindungsgemäße Zusammensetzungen, die die erfindungsgemäßen Copolymere enthalten, sind beispielsweise Seifen und Syndets.

Seife entsteht bei der Reaktion eines (Neutral-)Fettes oder daraus gewonnener Fettsäuren bzw. Fettsäuremethylester mit Natron- oder Kalilauge (Verseifung"). Seife ist in der Zusammensetzung chemisch das Alkalisalz von Fettsäuren. Als Neutralfette werden üblicherweise Rindertalg oder Palmöl im Gemisch mit Kokosöl oder Palmkernöl und - seltener - andere natürliche Öle oder Fette bei der Seifenherstellung eingesetzt, wobei die Qualität der Ausgangsfette stark mitbestimmend für die Güte der daraus gewonnenen Seife ist.

Wichtig für die Auswahl der Fettkomponenten ist die Verteilung der Kettenlängen der entsprechenden Fettsäuren. Gefragt sind normalerweise vor allem C12-C18-Fettsäuren. Da Lauratseife besonders gut schäumt, werden üblicherweise das laurinreiche Kokosöl oder das ähnlich aufgebaute Palmkernöl in höheren Anteilen (bis zu 50% des Neutralfettgemisches) für Seifen verwendet, bei denen viel Schaum bei der Benutzung erwünscht ist.

Die Natriumsalze der genannten Fettsäuregemische sind fest, die Kaliumsalze dagegen weich und pastös. Aus diesem Grunde wird als Laugenkomponente zur Herstellung fester Seifen vorzugsweise Natronlauge, für flüssigpastöse Seifen vorzugsweise Kalilauge verwendet. Bei der Verseifung wird das Verhältnis von Lauge zu Fettsäure so gewählt, dass allenfalls ein minimaler Überschuss an Lauge (max.0,05 %) im fertigen Seifenstück vorhanden ist.

Zu den Seifen werden üblicherweise Toilette-, Kern-, Transparent-, Luxus-, Creme-, Frische-/Deo-, Baby-, Hautschutz-, Abrasiv-, Schwimm- und Flüssigseifen sowie Waschpasten und Seifenblättchen gezählt.

Erfindungsgemäße Seifen enthalten neben den erfindungsgemäßen Copolymeren vorteilhafterweise weiterhin Antioxidantien, Komplexierungs- und Feuchthaltemittel sowie gegebenenfalls Duftstoffe, Farbstoffe und weitere, kosmetisch akzeptable Inhaltsstoffe. Solche weiteren geeigneten Inhaltsstoffe sind vorstehend genannt.

Syndets (von engl. synthetic detergent) sind Alternativen zu den herkömmlichen Seifen, die durch die gegenüber der Seife unterschiedliche Zusammensetzung gewisse Vorteile aufweist, wo Seife eher Nachteile besitzt.

Syndets enthalten als Schaum- und Reinigungskomponenten waschaktive Substanzen (Tenside), die durch chemische Synthese gewonnen werden. Seifen dagegen sind - wie beschrieben - Salze natürlich vorkommender Fettsäuren. Für Syndets werden hautmilde, biologisch gut abbaubare Tenside verwendet, vorzugsweise Fettsäure-Isethionate (Sodium Cocoyl Isethionate), Sulfobernsteinsäure-Halbester (Disodium Lauryl Sulfosuccinate), Alkylpolyglucoside (Decyl Glucoside), Amphotertenside (z. B. Sodium Cocoamphoacetate). Daneben spielen Monoglycerid-Sulfat und Ethercarboxylate vereinzelt eine Rolle. Fettalkoholsulfat (z. B. Sodium Lauryl Sulfate) hat seine einstige Bedeutung als Basistensid für Syndets weitestgehend verloren. Die Basistenside werden mit Gerüstsubstanzen, Rückfettungsmitteln und weiteren Zusätzen zu Formulierungen kombiniert, die sich nach üblicher Seifentechnologie verarbeiten lassen und Stücke ergeben, die sich möglichst "seifenähnlich", aber ohne die erwähnten Nachteile der Seife verhalten. Sie schäumen bei jeder Wasserhärte und besitzen eine sehr gute Reinigungskraft. Ihr pH-Wert ist in einem breiten Bereich (meist zwischen 4 und 8) einstellbar.

Aufgrund der intensiveren Reinigungs-/Entfettungskraft der Basistenside ist der Tensidanteil im Syndet gewöhnlich deutlich geringer, der Anteil an Überfettungsmitteln deutlich höher als in Seifen, ohne dass das Schaumvermögen herabgesetzt wird. Syndets werden speziell zur Reinigung der sensiblen Haut, der jugendlich-unreinen Haut und zur Gesichtswäsche empfohlen.

Neben den (seifenfreien) Syndets findet man auch das Marktsegment der Halb- oder Combars (abgeleitet von Combination bar). Dabei handelt es sich um Stücke, die sowohl Seife als auch Syndettenside enthalten. Combars enthalten 10 bis 80 Gew.-% Seife. Sie stellen für die Kriterien Kosten, Schaumvermögen, Hautgefühl und Verträglichkeit einen Kompromiss zwischen Seifen und Syndets dar. Beim Waschen mit einem Combar stellt sich, abhängig von seinem Seifenanteil, ein pH-Wert von ca. 7 bis 9 ein.

Hinsichtlich dem Fachmann bekannter, möglicher Rezepturen für Seifen und Syndets sei auf "Kosmetik und Hygiene von Kopf bis Fuß", Hrsg. W. Umbach, 3. Auflage, Wiley-VCH, 2004, S.112-122 verwiesen, worauf an dieser Stelle vollumfänglich Bezug genommen wird.

Duschbad- und Badeprodukte

Hinsichtlich spezieller Zusammensetzungen für Duschbad- und Badeprodukte oder Waschlotionen sei auf "Kosmetik und Hygiene von Kopf bis Fuß", Hrsg. W. Umbach, 3. Auflage, Wiley-VCH, 2004, S.128-134 verwiesen, worauf an dieser Stelle vollumfänglich Bezug genommen wird.

Ein weiterer Gegenstand der Erfindung ist die Verwendung eines erfindungsgemäßen Copolymers als Hilfsmittel in der Pharmazie, bevorzugt als oder in Beschichtungsmittel(n) für feste Arzneiformen, zur Modifizierung rheologischer Eigenschaften, als oberflächenaktive Verbindung, als oder in Klebemittel(n) sowie als oder in Beschichtungsmittel(n) für die Textil-, Papier-, Druck- und Lederindustrie.

Beispiele

Herstellbeispiele

Abkürzungen:

| | |
|---|---|
| VE: | voll entsalzt |
| TMAEMC: | 2-Trimethylammoniummethylmethacrylatchlorid |
| Quat311 | 2-Trimethylammoniummethylmethacrylatethylsulfat |
| VI: | N-Vinylimidazol |
| VP: | N-Vinylpyrrolidon |
| MAS: | Methacrylsäure |
| min: | Minute(n) |
| h: | Stunde(n) |
| Mon. | Monate |
| WS: | Wirkstoff; z.B. reines Polymer, ohne Lösungsmittel oder sonstige Beimischungen |

Die %-Angaben im Folgenden bedeuten Gew.-% sofern nicht anderweitig vermerkt. Die Angaben zur Monomerzusammensetzung wie beispielweise" 75/12,5/12,5" geben die Gewichtsverhältnisse der zur Polymerisation eingesetzten Monomere wieder.

Beispiel H1: TMAEMC/VI/VP 75/12,5/12,5

85,00 g VE-Wasser, 28,00 g 2-Trimethylammoniummethylmethacrylatchlorid (75 Gew.-%ig) und 0,60 g Mercaptoethanol wurden vorgelegt und mit 50 Gew.-% iger NaOH-Lösung auf pH = 8,5 gestellt. Die Vorlage wurde mit Stickstoff begast und unter Rühren auf 57°C aufgeheizt. Dann wurden1,98 g des Zulaufs 2 batch zugegeben und 15 min. anpolymerisiert. Der Zulauf 1 wurde innerhalb von 5 h zudosiert, während der Restzulauf 2 (26,37 g) innerhalb von 5,5 h zudosiert wurde. Nach Zulaufende wurde die Reaktionsmischung auf 70°C aufgeheizt und 1 h nachpolymerisiert. Nach dem Abkühlen wurde die Polymerlösung mit 1,90 g Phenonip konserviert.

Zulauf 1:

122,00 g 2-Trimethylammoniummethylmethacrylatchlorid (75 Gew.-%ig), 70,00 g VE-Wasser, 3,90 g Mercaptoethanol, 18,75 g N-Vinylimidazol, 18,75 g N-Vinylpyrrolidon mit 50 Gew.-%iger NaOH auf pH 8.5 gestellt

Zulauf 2:

26,25 g VE-Wasser, 2,10 g Wako®V 50

Beispiel H1b: TMAEMC/VI/VP 75/12,5/12,5

85,00 g VE-Wasser, 28,00 g 2-Trimethylammoniummethylmethacrylatchlorid (75 Gew.-%ig) und 0,60 g Mercaptoethanol wurden vorgelegt und mit 50 Gew.-% iger NaOH-Lösung auf pH = 8,5 gestellt. Die Vorlage wurde mit Stickstoff begast und unter Rühren auf 57 °C aufgeheizt. Dann wurden 1,98 g des Zulaufs 3 batch zugegeben und 15 min. anpolymerisiert. Zulauf 1 und Zulauf 2 wurden innerhalb von 5 Stunden zudosiert. Restzulauf 3 wurde innerhalb von 5,5 Stunden zudosiert. Nach Zulaufende wurde die Reaktionsmischung auf 70°C aufgeheizt und 1 Stunde nachpolymerisiert. Nach dem Abkühlen wurde die Polymerlösung mit 1,90 g Phenonip konserviert.

Zulauf 1:

122,00 g 2-Trimethylammoniumethylmethacrylatchlorid (75 Gew.-%ig), 70,00 g VE-Wasser, mit 50 Gew.-% iger NaOH-Lösung auf pH = 8,5 gestellt

Zulauf 2:

18,75 g N-Vinylimidazol, 18,75 g N-Vinylpyrrolidon, 1,65 g Mercaptoethanol

Zulauf 3:

2,70 g Wako®V50, 26,25 g VE-Wasser

Beispiel V1: TMAEMC/VP 75/25 (Vergleichsversuch)

85,00 g VE-Wasser, 28,00 g 2-Trimethylammoniumethylmethacrylatchlorid (75 Gew.-%ig) und 0,60 g Mercaptoethanol wurden vorgelegt und mit 50 Gew.-% iger NaOH-Lösung auf pH = 8,5 gestellt. Die Vorlage wurde mit Stickstoff begast und unter Rühren auf 57 °C aufgeheizt. Dann wurden 1.98 g des Zulaufs 2 batch zugegeben und 15 min. anpolymerisiert. Der Zulauf 1 wurde innerhalb von 5 h zudosiert, während der Restzulauf 2 (26,37 g) innerhalb von 5,5 h zudosiert wurde. Nach Zulaufende wurde die Reaktionsmischung auf 70 °C aufgeheizt und 1 h nachpolymerisiert. Nach dem Abkühlen wurde die Polymerlösung mit 1,90 g Phenonip konserviert.

Zulauf 1:

122,00 g 2-Trimethylammoniumethylmethacrylatchlorid (75 Gew.-%ig), 70,00 g VE-Wasser, 3,90 g Mercaptoethanol, 37,50 g N-Vinylpyrrolidon wurden mit 50 Gew.-%iger NaOH auf pH 8.5 gestellt

Zulauf 2:

26,25 g VE-Wasser, 2,10 g Wako®V 50

Beispiel V2 : Quat311/VI/VP 82/9/9 (Vergleichsversuch)

80,00 g VE-Wasser, 134,84 g von Zulauf 1 und 15,00 g von Zulauf 2 wurden vorgelegt, mit Stickstoff begast und auf 75 °C aufgeheizt. Bei Erreichen dieser Temperatur wurden 20,54 g des Zulaufs 3 zugegeben und 15 min anpolymerisiert. Die Restzuläufe 1 und 2 wurden dann über 5 Stunden zudosiert und Restzulauf 3 über 5,5 Stunden. Nach Zulaufende wurde 1 Stunde nachpolymerisiert, abgekühlt und 5,20 g Phenonip eingerührt.

Zulauf 1:

674,20 g Quat311 (50% ig)

Zulauf 2:

37,50 g N-Vinylpyrrolidon, 37,50 g N-Vinylimidazol

Zulauf 3:

200,00 g VE-Wasser, 5,40 g Wako®V50

Beispiel H2: TMAEMC/VI/VP 75/12,5/12,5

325.85 g VE-Wasser, 150,00 g 2-Trimethylammoniumethylmethacrylatchlorid (75 Gew.-%ig), 18,75 g N-Vinylimidazol, 18,75 g N-Vinylpyrrolidon und 62,00 g Isopropanol wurden vorgelegt, mit Stickstoff begast und unter Rühren auf 75°C aufgeheizt.

Dann wurden 3.34 g des Zulaufs 1 auf einmal zugegeben und es wurde 15 min anpolymerisiert. Der Restzulauf 1 (44.36 g) wurde daraufhin innerhalb 3.5 h zudosiert. Nach Zulaufende wurde 1 h nachpolymerisiert und im Anschluß wasserdampfdestilliert. Nach dem Abkühlen wurde die Polymerlösung mit 3.15 g Phenonip konserviert.

Zulauf 1:

2,70 g Wako®V 50 und 45,00 g VE-Wasser

Beispiel H3: TMAEMC/VI/VP/MAS 75/9/9/7

541,50 g VE-Wasser, 150,00 g 2-Trimethylammoniumethylmethacrylatchlorid (75 Gew.-%ig), 13,50 g N-Vinylimidazol und 10, 50 g Methacrylsäure wurden vorgelegt und mit 50 Gew.-%iger NaOH-Lösung auf pH = 8,5 eingestellt. Dann wurden 13.50 g N-Vinylpyrrolidon zugegeben und die Reaktionsmischung unter Rühren mit Stickstoff begast und auf 80°C aufgeheizt. Währenddessen wurden 3,30 g des Zulaufs 1 auf einmal zugegeben und es wurde 15 min. anpolymerisiert. Die Restmenge des Zulaufs 1 (43,8 g) wurde innerhalb von 2,5 h zudosiert und nach Zulaufende noch 1 h nachpolymerisiert.

Nach dem Abkühlen wurde die Polymerlösung mit 3,90 g Phenonip konserviert.

Zulauf 1:

2,10 g Wako®V50 und 45,00 g VE Wasser

Beispiel H3b: TMAEMC/VI/VP/MAS 75/9/9/7

541,50 g VE-Wasser, 150,00 g 2-Trimethylammoniumethylmethacrylatchlorid (75 Gew.-%ig), 13,50 g N-Vinylimidazol und 10, 50 g Methacrylsäure wurden vorgelegt und mit 50 Gew.-%iger NaOH-Lösung auf pH = 7,0 eingestellt. Danach wurden 13.50 g N-Vinylpyrrolidon zugegeben, der pH-Wert erneut überprüft, die Reaktionsmischung unter Rühren mit Stickstoff begast und auf 80°C aufgeheizt. Dann wurden 3,30 g des Zulaufs 1 batch zugegeben und es wurde 15 min. anpolymerisiert. Die Restmenge des Zulaufs 1 (43,8 g) wurde innerhalb von 2,5 Stunden zudosiert und nach Zulaufende noch 1 Stundenachpolymerisiert. Nach dem Abkühlen wurde die Polymerlösung mit 3,90 g Phenonip konserviert.

Zulauf 1:

2,10 g Wako®V50 und 45,00 g VE Wasser

Beispiel H3c: TMAEMC/VI/VP/MAS 75/9/9/7

541,50 g VE-Wasser, 150,00 g 2-Trimethylammoniumethylmethacrylatchlorid (75 Gew.-%ig), 13,50 g N-Vinylimidazol und 10, 50 g Methacrylsäure wurden vorgelegt und mit 50 Gew.-%iger NaOH-Lösung auf pH = 6,0 eingestellt. Danach wurden 13.50 g N-Vinylpyrrolidon zugegeben, der pH-Wert erneut überprüft, die Reaktionsmischung unter Rühren mit Stickstoff begast und auf 80°C aufgeheizt. Dann wurden 3,30 g des Zulaufs 1 batch zugegeben und es wurde 15 min. anpolymerisiert. Die Restmenge des Zulaufs 1 (43,8 g) wurde innerhalb von 2,5 Stunden zudosiert und nach Zulaufende noch 1 Stunde nachpolymerisiert. Nach dem Abkühlen wurde die Polymerlösung mit 3,90 g Phenonip konserviert.

Zulauf 1:

2,10 g Wako®V50 und 45,00 g VE Wasser

Beispiel H4: TMAEMC/VI/VP 75/10/15

185,70 g VE-Wasser, 115,00 g 2-Trimethylammoniumethylmethacrylatchlorid (75 Gew.-%ig), 11,50 g N-Vinylimidazol, 17,25 g N-Vinylpyrrolidon und 109,40 g Isopropanol wurden vorgelegt, mit Stickstoff begast und unter Rühren auf 75°C aufgeheizt. Dann wurden 2,56 g des Zulaufs 1 batch zugegeben und es wurde 15 min anpolymerisiert. Der Restzulauf 1 (34,01 g) wurde daraufhin innerhalb 5,5 h zudosiert.

Nach Zulaufende wurde 1 h nachpolymerisiert und im Anschluß wasserdampfdestilliert. Nach dem Abkühlen wurde die Polymerlösung mit 2,38 g Phenonip konserviert.

Zulauf 1:

2,07 g Wako®V 50 und 34,50 g VE-Wasser

Beispiel H4b: TMAEMC/VI/VP 75/10/15

417,03 g VE-Wasser, 115,00 g 2-Trimethylammoniumethylmethacrylatchlorid (75 Gew.-%ig), 11,50 g N-Vinylimidazol und 17,25 g N-Vinylpyrrolidon wurden vorgelegt, mit Stickstoff begast und unter Rühren auf 80°C aufgeheizt.
Dann wurden 2,56 g des Zulaufs 1 batch zugegeben und es wurde 15 min anpolymerisiert. Der Restzulauf 1 (34,01 g) wurde daraufhin innerhalb 2,5 h zudosiert.
Nach Zulaufende wurde 1 h nachpolymerisiert und nach dem Abkühlen wurde die Polymerlösung mit 2,38 g Phenonip konserviert.

Zulauf 1:

2,07 g Wako®V 50 und 34,50 g VE-Wasser

Beispiel H5 : TMAEMC/VI/VP 80/5/15

400,00 g VE-Wasser und 160,00 g 2-Trimethylammoniumethylmethacrylatchlorid (75 Gew.-%ig) wurden in die Vorlage gegeben, mit Stickstoff begast und auf 75°C aufgeheizt. Bei dieser Temperatur wurden dann 6 g des Zulaufs 1 und 2 g des Zulaufs 2 batch zugegeben und es wurde 15 min anpolymerisiert. Der Restzulauf 1 wurde innerhalb von 5 Stunden und der Restzulauf 2 innerhalb von 5,5 Stunden zudosiert. Nach Zulaufende wurde 1 Stunde nachpolymerisiert und nach dem Abkühlen mit 1,90 g Phenonip konserviert.

Zulauf 1:

22,50 g N-Vinylpyrrolidon, 7,50 g N-Vinylimidazol

Zulauf 2:

2.10 g Wako®V 50, 26,25 g VE-Wasser

Beispiel H6: TMAEMC/VI/VP 80/12.5/7.5

385,05 g VE-Wasser, 160,00 g 2-Trimethylammoniumethylmethacrylatchlorid (75 Gew.-%ig), 18,75 g N-Vinylimidazol und 11,25 g N-Vinylpyrrolidon wurden vorgelegt, mit Stickstoff begast und unter Rühren auf 75°C aufgeheizt.
Dann wurden 3,34 g des Zulaufs 1 batch zugegeben und es wurde 15 min anpolymerisiert. Der Restzulauf 1 (44,36 g) wurde daraufhin innerhalb 5,5 h zudosiert.
Nach Zulaufende wurde 1 h nachpolymerisiert. Nach dem Abkühlen wurde die Polymerlösung mit 3,15 g Phenonip konserviert.

Zulauf 1:

2,70 g Wako®V 50 und 45,00 g VE-Wasser

Beispiel H7: TMAEMC/VI/VP 80/10/10

385,05 g VE-Wasser, 160,00 g 2-Trimethylammoniumethylmethacrylatchlorid (75 Gew.-%ig), 15,00 g N-Vinylimidazol und 15,00 g N-Vinylpyrrolidon wurden vorgelegt, mit Stickstoff begast und unter Rühren auf 75°C aufgeheizt.
Dann wurden 3,34 g des Zulaufs 1 batch zugegeben und es wurde 15 min anpolymerisiert. Der Restzulauf 1 (44,36 g) wurde daraufhin innerhalb 5,5 h zudosiert.
Nach Zulaufende wurde 1 h nachpolymerisiert. Nach dem Abkühlen wurde die Polymerlösung mit 3,15 g Phenonip konserviert.

Zulauf 1:

2,70 g Wako®V 50 und 45,00 g VE-Wasser

Beispiel H8: TMAEMC/VI/VP 78/13.2/8.8

172,66 g VE-Wasser, 156,00 g 2-Trimethylammoniumethylmethacrylatchlorid (75 Gew.-%ig), 13,20 g N-Vinylimidazol, 19,80 g N-Vinylpyrrolidon und 74,48 g Isopropanol wurden vorgelegt, mit Stickstoff begast und unter Rühren auf 70°C aufgeheizt.

Dann wurden 3,34 g des Zulaufs 1 batch zugegeben und es wurde 15 min anpolymerisiert. Der Restzulauf 1 (44,36 g) wurde daraufhin innerhalb von 4,0 h zudosiert.

Nach Zulaufende wurde auf 75°C aufgeheizt und 0,45g Wako®V50 in 4,00 g VE-Wasser batch zugegeben und 1 h nachpolymerisiert. Im Anschluß wurde wasserdampfdestilliert.

Nach dem Abkühlen wurde die Polymerlösung mit 2,45 g Phenonip konserviert.

Zulauf 1:

2,70 g Wako®V 50 und 45,00 g VE-Wasser

Anwendungstechnische Prüfungen

Bestimmung der K-Werte

Die K-Werte wurden nach Fikentscher, Cellulosechemie, Bd. 13, S. 58 bis 64 (1932) bei 25°C in wässriger Lösung gemessen und stellen ein Maß für das Molgewicht dar. Die Lösung der Polymere enthält 1 g Polymer in 100 ml Lösung.

Die Messung des K-Wertes erfolgt in einer Micro-Ubbelohde-Kapillare Typ M Ic der Fa. Schott.

Nasskämmbarkeit (europäische, gebleichte Haartressen):

Bestimmung Blindwert

Vor der Bestimmung wurde die gebleichte Haartresse (Länge ca. 24 cm / Gewicht 2,7-3,3 g) zunächst zweimal mit Texapon®NSO insgesamt 1 Minute shampooniert und 1 Minute ausgespült um eine definierte Nässe und Quellung zu erreichen.

Danach wurde die Tresse so vorgekämmt, bis keine Verhakungen der Haare mehr vorhanden waren.

Anschließend wurde die Tresse an der Halterung fixiert und mit der feinzinkigen Seite des Kammes in die feinzinkige Seite des Prüfkammes eingekämmt. Das Einlegen der Haare in den Prüfkamm erfolgte bei jeder Messung gleichmäßig und spannungsfrei. Die Messung wurde gestartet und mit Hilfe der Software EGRANUDO® (Fa. Frank) ausgewertet. Die Messung wurde 5-10 mal wiederholt. Die Messungen wurden im Klimaraum bei ca. 65% relativer Feuchte und 21 °C durchgeführt.

Der errechnete Mittelwert wurde zusammen mit der Standardabweichung notiert.

Shampoo-Rezeptur:

| | |
|---|---|
| 35,70g | Texapon®NSO |
| 12,50g | Tego Betain L 7 |
| 0,5 g | erfindungsgemäßes Copolymer effektiv (0,5 g gerechnet als Feststoff) |
| 0,10g | Euxyl®K 100 |
| ad 100g | Wasser |
| 1,00g | NaCl |

5 g des zu testenden Shampoos wurden aufgetragen, 1 Min. shampooniert, 1 Min. ausgespült, auf Filterpapier abgedrückt und gekämmt und der Messwert bestimmt.

Auswertung:

$$\text{Kämmkraftabnahme nass} = 100 - (\text{Messwert} * 100 / \text{Blindwert}); \text{ Angabe in \%}$$

Angabe in % Verwendete Geräte: Zug/ Druck-Prüfgerät der Fa. Frank

Digitalwaage (Oberschalenwaage)

Griff

Während des Shampooniervorganges von 1 Minute (siehe oben Bestimmung Messwert Nasskämmbarkeit) wird das Aufschäumverhalten, die Schaumcremigkeit, das Pflegeverhalten und das Schaumvolumen beurteilt. Danach wird die Tresse 1 Minute unter fließendem Trinkwasser (Duschbrause) ausgespült. Mit einer Hand wird die Haartresse oben an der Naht unter die Duschbrause gehalten, mit der anderen Hand lässt man die Haare zwischen Daumen und Handinnenfläche von oben nach unten entlang gleiten. Durch das Tragen von Gummihandschuhen spürt man beim Entlanggleiten, welchen Griff die Haare aufweisen.
Es erfolgt die subjektive Beurteilung des wachsigen Griffs (Silicongriff) der nassen Haare

- kein wachsiger Griff, (wie unbehandeltes Haar) bremsend (+) entspricht "schlecht"
- leicht wachsig, leichtes gleiten (++) entspricht "gut"
- wachsig, sehr leichtes gleiten (+++) entspricht "sehr gut"

Die ausgespülte Haartresse wird zwischen Mittel und Zeigefinger abgestreift, auf Filterpapier abgedrückt, gekämmt und die Apparatur eingespannt.
Beim Einkämmen in die Kämmvorrichtung sind Unterschiede festzustellen d.h. je wachsiger der Griff am Haar, um so geringer ist der Widerstand beim Einkämmen in den Prüfkamm. Die Messung der Kämmkraft erfolgt analog der Blindwertbestimmung.

Zentrifugentest:

12 g des Shampoos mit 0.5 Gew.-% Polymer werden in einem Zentrifugenglas 15 Minuten bei ca. 4700 Upm und 20°C zentrifugiert (Zentrifuge Multifuge 1S-R (HERAEUS). Es erfolgt danach eine augenscheinliche visuelle Beurteilung. Beobachtung eines Niederschlages oder Bodensatzbildung bedeutet, dass die Formulierung nicht stabil ist.

Lagerstabilität des Shampoos:

Ein nach der oben genannten Rezeptur hergestelltes Shampoo (0,5 % Wirkstoff) wurde 3 Monate bei 40°C gelagert. Jeweils nach 2 und 6 Wochen und nach 3 Monaten wurden die Shampoos begutachtet und auf etwaige Ausfällungen hin untersucht.

| Bsp. | PolymerZusammensetzung | Feststoffgehalt % | K-Wert | 0,2% WS leicht wachsiger Griff ++ 0,5% WS wachsiger Griff +++ | Stabilität Shampoo (40°C) | Nasskämmbarkeit Handnote | Nasskämmbarkeit Messwert |
|---|---|---|---|---|---|---|---|
| V1 | TMAEMC/VP 75/25 | 42,8 | 43,4 | nicht vorh. | Zentrifugentest stabil | 2+ | 44%±4 (0,2% WS) |
| V2 | Quat311/VI/VP 82/9/9 | 44,1 | 37,3 | nicht vorh. | Zentrifugentest stabil | 1-2 | 61%±3 (0,5% WS) |
| H1 | TMAEMC/VI/VP 75/12,5/12,5 | 43,8 | 57 | 0,2%: ++ | 3 Monate: noch stabil | 1- | 72%±1 (0,2% WS) |
| H1b | TMAEMC/VI/VP 75/12,5/12,5 | 48,5 | 50,3 | 0,5%: ++ | Zentrifugentest stabil | 1 | 74%±2 (0,5% WS) |
| H2 | TMAEMC/VI/VP 75/12,5/12,5 | 24,3 | 64 | 0,5%: +++ Wachsgriff schon beim Ausspülen | 3 Mon. stabil, sehr leichter Niederschlag | 1 | 80%±2; 82%±2 (0,5% WS) |
| H3 | TMAEMC/VI/VP/MAS 75/9/9/7 | 21,4 | 71,2 | 0,5%: +++ Wachsgriff erst beim Entwirren und Einkämmen | Zentrifugentest stabil | 1 | 76%±2 (0,5% WS) |
| H3b | TMAEMC/VI/VP/MAS 75/9/9/7 | 21,0 | 74,6 | 0,5%: +++ Wachsgriff schon beim Ausspülen | 3 Mon. noch stabil, vereinzelte Partikel, Belag | 1 | 80%±2 (0,5% WS) |
| H3c | TMAEMC/VI/VP/MAS 75/9/9/7 | 19,55 | 75,2 | 0,5%: +++ Wachsgriff schon beim Ausspülen | 3 Mon. noch stabil, sehr leichter Niederschlag, vereinzelte Partikel | 1 | 76%±3 (0,5% WS) |
| H4 | TMAEMC/VI/VP 75/10/15 | 24,7 | 51,6 | 0,5%: +++ Wachsgriff schon beim Ausspülen | 6 Wochen stabil | 1 | 83%±2; 85%±2 (0,5% WS) |
| H4b | TMAEMC/VI/VP 75/10/15 | 21.0 | 81.9 | 0,5%:++(+) Wachsgriff schon beim Ausspülen | 3 Mon. stabil, Belag am Glasboden | 1 | 76%±3 (0,5% WS) |
| H5 | TMAEMC/VI/VP 80/5/15 | 26,7 | 54,5 | 0,5%: +++ Wachsgriff schon beim Ausspülen | 6 Wochen stabil | 1 | 82%±1 (0,5% WS) |
| H6 | TMAEMC/VI/VP 80/12.5/7.5 | 26.5 | 66.3 | 0,5%: +++ Wachsgriff schon beim Ausspülen | 3 Mon. stabil, leichter Niederschlag | 1 | 76%±3 (0,5% WS) |
| H7 | TMAEMC/VI/VP 80/10/10 | 26.2 | 73.8 | 0,5%: +++ Wachsgriff erst beim Entwirren und Einkämmen | 3 Mon. stabil, leichter Niederschlag | 1 | 76%±3 (0,5% WS) |
| H8 | TMAEMC/VI/V 78/13.2/8.8 | 31.9 | 63.3 | 0,5%: +++ Wachsgriff schon beim Ausspülen | 3 Mon. stabil, leichter Bodensatz | 1 | 77%±3 (0,5% WS) |

Shampooformulierungen / Duschgelformulierungen

Bevorzugte Shampooformulierungen oder Duschgelformulierungen enthalten

a) 0,01 bis 5 Gew.-% eines erfindungsgemäßen Copolymers
b) 25 bis 99,99 Gew.-% Wasser
c) 0 - 5 Gew.-% eines weiteren Konditioniermittels
d) 0 - 30 Gew.-% weitere kosmetische Bestandteile

In den Shampooformulierungen können zudem alle in Shampoos üblicherweise eingesetzten anionischen, neutralen, amphoteren oder kationischen Tenside verwendet werden mit den vorstehenden Maßgaben.

Beispiel 1: Conditioner Shampoo mit PQ-10

| | |
|---|---|
| 35,70 g | Sodium Laureth Sulfate |
| 6,50 g | Cocamidopropyl Betaine |
| 0,20 g | Copolymer gemäß Beispiel H1 |
| 0,40 g | Polyquaternium-10 |
| 0,10 g | Konservierungsmittel |
| 0,10 g | Parfumöl / etherisches Öl |
| ad 100 g | Aqua dem. |

Gute Conditioner Shampoos werden auch erhalten, wenn anstelle des Copolymers gemäß Beispiel H1 eines oder mehrere der Copolymere H 1 b, H2, H3, H3b, H3c, H4, H4b, H5, H6, H7 oder H8 verwendet werden.

Beispiel 2: Conditioner Shampoo mit GHTC

| | |
|---|---|
| 35,70 g | Sodium Laureth Sulfate |
| 6,50 g | Cocamidopropyl Betaine |
| 0,50 g | Copolymer gemäß Beispiel H1 |
| 0,20 g | Guarhydroxypropyltrimonium Chloride |
| 0,10 g | Konservierungsmittel |
| 0,10 g | Parfumöl / etherisches Öl |
| ad 100 g | Aqua dem. |

Gute Conditioner Shampoos werden auch erhalten, wenn anstelle des Copolymers gemäß Beispiel H1 eines oder mehrere der Copolymere H 1 b, H2, H3, H3b, H3c, H4, H4b, H5, H6, H7 oder H8 verwendet werden.

Beispiel 3: Conditioner Shampoo mit Polyquaternium

| | |
|---|---|
| 35,70 g | Sodium Laureth Sulfate |
| 6,50 g | Cocamidopropyl Betaine |
| 0,20 g | Copolymer gemäß Beispiel H1 |
| 0,30 g | Polyquaternium-44 oder Polyquaternium-67 |
| 0,10 g | Konservierungsmittel |
| 0,10 g | Parfumöl / etherisches Öl |
| ad 100 g | Aqua dem. |

Gute Conditioner Shampoos werden auch erhalten, wenn anstelle des Copolymers gemäß Beispiel H1 eines oder mehrere der Copolymere H 1 b, H2, H3, H3b, H3c, H4, H4b, H5, H6, H7 oder H8 verwendet werden.

Beispiel 4: Shampoo

| | Phase A |
|---|---|
| 15,00 g | Cocamidopropyl Betaine |
| 10,00 | Disodium Cocoamphodiacetate |
| 5,00 g | Polysorbate 20 |
| 5,00 g | Decyl Glucoside |
| 0,20 g | Copolymer gemäß Beispiel H1 |
| 0,10 g | Parfumöl / etherisches Öl |
| q.s. | Konservierungsmittel |
| 2,00 g | Laureth-3 |
| ad 100 | Aqua dem. |
| q.s. | Citric Acid |

| | Phase B |
|---|---|
| 3,00 g | PEG-150 Distearate |

Herstellung

Komponenten der Phase A einwiegen und lösen; pH-Wert auf 6-7 einstellen. Phase B zugeben und auf 50°C erwärmen. Auf Raumtemperatur abkühlen lassen unter Rühren. Gute Shampoos werden auch erhalten, wenn anstelle des Copolymers gemäß Beispiel H1 eines oder mehrere der Copolymere H1b, H2, H3, H3b, H3c, H4, H4b, H5, H6, H7 oder H8 verwendet werden.

Beispiel 5: Shampoo

| | |
|---|---|
| 30,00 g | Sodium Laureth Sulfate |
| 6,00 g | Sodium Cocoamphoacetate |
| 0,50 g | Copolymer gemäß Beispiel H1 |
| 3,00 g | Sodium Laureth Sulfate, Glycol Distearate, Cocamide MEA, Laureth-10 |
| 2,00 g | Dimethicone |
| q.s. | Parfum |
| q.s. | Konservierungsmittel |
| q.s. | Citric Acid |
| 1,00 g | Sodium Chloride |
| add 100 | Aqua dem. |

Gute Shampoos werden auch erhalten, wenn anstelle des Copolymers gemäß Beispiel H1 eines oder mehrere der Copolymere H1b, H2, H3, H3b, H3c, H4, H4b, H5, H6, H7 oder H8 verwendet werden.

Beispiel 6: Duschgel

| | |
|---|---|
| 20,00 g | Ammonium Laureth Sulfate |
| 15,00 g | Ammonium Lauryl Sulfate |

(fortgesetzt)

| | |
|---|---|
| 0,50 g | Copolymer gemäß Beispiel H1 |
| 0,50 g | Polyquaternium-7 |
| 2,50 g | Sodium Laureth Sulfate, Glycol Distearate, Cocamide MEA, Laureth-10 |
| 0,10 g | Parfumöl / etherisches Öl |
| q.s. | Konservierungsmittel |
| 0,50 g | Sodium Chloride |
| add 100 | Aqua dem. |

Gute Duschgele werden auch erhalten, wenn anstelle des Copolymers gemäß Beispiel H1 eines oder mehrere der Copolymere H1b, H2, H3, H3b, H3c, H4, H4b, H5, H6, H7 oder H8 verwendet werden.

Beispiel 7: Duschgel

| | |
|---|---|
| 40,00 g | Sodium Laureth Sulfate |
| 5,00 g | Decyl Glucoside |
| 5,00 g | Copolymer gemäß Beispiel H1 |
| 1,00 g | Panthenol |
| 0,10 g | Parfumöl / etherisches Öl |
| q.s. | Konservierungsmittel |
| q.s. | Citric Acid |
| 2,00 g | Sodium Chloride |
| add 100 | Aqua dem. |

Gute Duschgele werden auch erhalten, wenn anstelle des Copolymers gemäß Beispiel H1 eines oder mehrere der Copolymere H1b, H2, H3, H3b, H3c, H4, H4b, H5, H6, H7 oder H8 verwendet werden.

Beispiel 8: Shampoo

| | |
|---|---|
| 12,00 g | Sodium Laureth Sulfate |
| 1,50 g | Decyl Glucoside |
| 0,50 g | Copolymer gemäß Beispiel H1 |
| 5,00 g | Coco-Glucoside Glyceryl Oleate |
| 2,00 g | Sodium Laureth Sulfate, Glycol Distearate, Cocamide MEA, Laureth-10 |
| q.s. | Konservierungsmittel |
| q.s. | Sunset Yelow C. I. 15 985 |
| 0,10 g | Parfumöl / etherisches Öl |
| 1,00 g | Sodium Chloride |
| add 100 | Aqua dem. |

Gute Shampoos werden auch erhalten, wenn anstelle des Copolymers gemäß Beispiel H1 eines oder mehrere der Copolymere H1b, H2, H3, H3b, H3c, H4, H4b, H5, H6, H7 oder H8 verwendet werden.

Die erfindungsgemäßen Copolymere eignen sich auch in Haarstyling-Zubereitungen, insbesondere Haarschäume (Aerosolschäume mit Treibgas und Pumpschäume ohne Treibgas), Haarsprays (Pumpssprays ohne Treibgas) und Haargele.

Treibmittel sind die üblich verwendeten Treibmittel. Bevorzugt sind Gemische aus Propan/Butan, Pentan, Dimethylether, 1,1-Difluorethan (HFC-152 a), Kohlendioxid, Stickstoff oder Druckluft.

Aerosolhaarschaum

a) 0,1 bis 10 Gew.-% eines Kosmetik-Polymers
b) 55 bis 99,8 Gew.-% Wasser und/oder Alkohol
c) 5 bis 20 Gew.-% eines Treibmittels
d) 0,1 bis 5 Gew.-% eines erfindungsgemäßen Copolymers
e) 0 bis 10 Gew.-% weitere Bestandteile

Als weitere Bestandteile können unter anderem alle in Haarschäumen üblicherweise eingesetzten Emulgatoren verwendet werden. Geeignete Emulgatoren können nichtionisch, kationisch bzw. anionisch oder amphoter sein.
Beispiele für nichtionische Emulgatoren (INCI-Nomenklatur) sind Laurethe, z.B. Laureth-4; Cetethe, z.B. Cetheth-1, Polyethylenglycolcetylether; Cetearethe, z.B. Cetheareth-25, Polyglycolfettsäureglyceride, hydroxyliertes Lecithin, Lactylester von Fettsäuren, Alkylpolyglycoside.
Beispiele für kationische Emulgatoren sind Cetyldimethyl-2-hydroxyethyl-ammoniumdihydrogenphosphat, Cetyltrimoniumchlorid, Cetyltrimmoniumbromid, Cocotrimoniummethylsulfat, Quaternium-1 bis x (INCI).
Anionische Emulgatoren können beispielsweise ausgewählt werden aus der Gruppe der Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Beispiel 9: Aerosolhaarschaum

| | |
|---|---|
| 2.00 g | Cocotrimonium Methosulfate |
| 0,10 g | Parfumöl / etherisches Öl |
| 3,50 g | Festigerpolymer z.B. Polyquaternium-46, PQ-44, VP/Methacrylamide/ Vinyl Imidazole Copolymer, etc. |
| 0,80 g | Copolymer gemäß Beispiel H1 |
| q.s. | Preservative |
| 75,00 g | Wasser dem. |
| 10,00 g | Propane/Butane (3.5 bar) |

Gute Aerosolhaarschäume werden auch erhalten, wenn anstelle des Copolymers gemäß Beispiel H1 eines oder mehrere der Copolymere H1b, H2, H3, H3b, H3c, H4, H4b, H5, H6, H7 oder H8 verwendet werden.
Eine erfindungsgemäß für Styling-Gele geeignete Zubereitung kann beispielsweise wie folgt zusammengesetzt sein:

Stylinggel

a) 0,1 bis 10 Gew.-% eines Kosmetik-Polymers
b) 60 bis 99,85 Gew.-% Wasser und/oder Alkohol
c) 0,05 bis 10 Gew.-% eins Gelbildners
d) 0,1 bis 5 Gew.-% eines erfindungsgemäßen Copolymers
e) 0 bis 20 Gew.-% weitere Bestandteile

Als Gelbildner können alle in der Kosmetik üblichen Gelbildner eingesetzt werden.
Hierzu zählen leicht vernetzte Polyacrylsäure, beispielsweise Carbomer (INCI), Cellulosederivate, z.B. Hydroxypropylcellulose, Hydroxyethylcellulose, kationisch modifizierte Cellulosen, Polysaccharide, z.B. Xanthum Gummi, Caprylic/Capric Triglyceride, Sodium acrylates Copolymer, Polyquaternium-32 (and) Paraffinum Liquidum (INCI), Sodium Acrylates Copolymer (and) Paraffinum Liquidum (and) PPG-1 Trideceth-6, Acrylamidopropyl Trimonium Chloride/Acrylamide Copolymer, Steareth-10 Allyl Ether Acrylates Copolymer, Polyquaternium-37 (and) Paraffinum Liquidum (and) PPG-1 Trideceth-6, Polyquaternium-37 (and) Propylene Glycole Dicaprate Dicaprylate (and) PPG-1 Trideceth-6, Polyquaternium-7, Polyquaternium-44, Polyquaternium-67.

Gute Stylinggele werden auch erhalten, wenn anstelle des Copolymers gemäß Beispiel H1 eines oder mehrere der Copolymere H1b, H2, H3, H3b, H3c, H4, H4b, H5, H6, H7 oder H8 verwendet werden.

Beispiel 10: Haarstylinggel

| | |
|---|---|
| Phase A | |
| 0,50 g | Carbomer oder Acrylates/C10-30 Alkyl Acrylate Crosspolymer |
| 86,40 g | Wasser dem. |
| Phase B | |
| 0,70 g | Triethanolamine |
| Phase C | |
| 6,00 g | Festigerpolymer z.B. VP/Methacrylamide/Vinyl Imidazole Copolymer |
| 5,00g | PVP |
| 0,20 g | PEG-25 PABA |
| 0,50 g | Copolymer gemäß Beispiel H1 |
| 0,10 g | Parfumöl / etherisches Öl |
| q.s. | PEG-14 Dimethicone |
| q.s. | Konservierungsmittel |
| 0,10 g | Tocopheryl Acetate |

Gute Stylinggele werden auch erhalten, wenn anstelle des Copolymers gemäß Beispiel H1 eines oder mehrere der Copolymere H1b, H2, H3, H3b, H3c, H4, H4b, H5, H6, H7 oder H8 verwendet werden.

Beispiel 11: Haarstylinggel

| | |
|---|---|
| Phase A | |
| 0,50 g | Carbomer oder Acrylates/C10-30 Alkyl Acrylate Crosspolymer |
| 91,20 g | Wasser dem. |
| Phase B | |
| 0,90 g | Tetrahydroxypropyl Ethylenediamine |
| Phase C | |
| 7,00 g | VP/VA Copolymer |
| 0,40 g | Copolymer gemäß Beispiel H1 |
| 0,20 g | Parfumöl / etherisches Öl |
| q.s. | Konservierungsmittel |
| 0,10 g | Propylene Glycol |

Gute Stylinggele werden auch erhalten, wenn anstelle des Copolymers gemäß Beispiel H1 eines oder mehrere der Copolymere H1b, H2, H3, H3b, H3c, H4, H4b, H5, H6, H7 oder H8 verwendet werden.

Beispiel 12: Hair Wax Cream

| | |
|---|---|
| 6,00 g | Caprylic/Capric Triglyceride |
| 3,00 g | Glyceryl Stearate |
| 2,00 g | Cetyl Alcohol |
| 3,50 g | Copolymer gemäß Beispiel H1 |
| 0,50 g | Cremophoer A6 |
| 0,70 g | Cremophor A25 |
| 0,50 g | Dimethicone |
| 0,50 g | Vitamin E Acetate |
| 2,00 g | Caprylic/Capric Triglyceride and Sodiumacrylates Copolymer |
| 1,00 g | D-Panthenol USP |
| 0,10 g | EDTA |
| 10,00 g | Festigerpolymer |
| q.s. | Konservierungsmittel |
| ad 100 g | Wasser dem. |

Gute Hair Wax Creams werden auch erhalten, wenn anstelle des Copolymers gemäß Beispiel H1 eines oder mehrere der Copolymere H1b, H2, H3, H3b, H3c, H4, H4b, H5, H6, H7 oder H8 verwendet werden.

Beispiel 13: Haarpudding

| | |
|---|---|
| 3,00 g | Kollicoat IR (BASF) |
| q.s. | Konservierungsmittel |
| 2,00 g | Festigerpolymer |
| 4,00 g | Acrylates/beheneth-25 Methacrylate Copolymer |
| 0,70 g | Copolymer gemäß Beispiel H1 |
| 0,50 g | Dimethicone Copolyol |
| 0,10 g | EDTA |
| 0,20 g | Benzophenone-4 |
| ad 100 g | Wasser dem. |

Gute Haarpuddings werden auch erhalten, wenn anstelle des Copolymers gemäß Beispiel H1 eines oder mehrere der Copolymere H1b, H2, H3, H3b, H3c, H4, H4b, H5, H6, H7 oder H8 verwendet werden.

Beispiel 14: Sprühgel

| | |
|---|---|
| Phase A | |
| 1,25 g | Festigerpolymer |
| 96,15 g | Aqua dem. |
| Phase B | |
| 0,70 g | Acrylates/Steareth-20 Itaconate Copolymer |
| 0,10 g | Propylene Glycol |
| 0,50 g | Copolymer gemäß Beispiel H1 |
| 0,10 g | Glycerin |
| 0,10 g | Parfumöl / etherisches Öl |

(fortgesetzt)

| | |
|---|---|
| Phase B | |
| q.s. | Konservierungsmittel |
| Phase C | |
| 0,70 g | Triethanolamine |

Gute Sprühgele werden auch erhalten, wenn anstelle des Copolymers gemäß Beispiel H1 eines oder mehrere der Copolymere H1b, H2, H3, H3b, H3c, H4, H4b, H5, H6, H7 oder H8 verwendet werden.

Eine erfindungsgemäß für Styling-Sprays geeignete Zubereitung kann beispielsweise wie folgt zusammengesetzt sein:

Beispiel 15: Pumphaarspray

| | |
|---|---|
| 11,20 g | PEG/PPG-25/25 Dimethicone/Acrylates Copolymer |
| 2,80 g | VP/VA Copolymer |
| 1,34 g | Aminomethyl Propanol |
| 0,30 g | Copolymer gemäß Beispiel H1 |
| 0,10 g | Parfumöl / etherisches Öl |
| 11,26 g | Aqua dem. |
| 73,00 g | Alcohol |

Gute Pumphaarsprays werden auch erhalten, wenn anstelle des Copolymers gemäß Beispiel H1 eines oder mehrere der Copolymere H1b, H2, H3, H3b, H3c, H4, H4b, H5, H6, H7 oder H8 verwendet werden.

Beispiel 16: Pumphaarspray VOC55

| | |
|---|---|
| 2,00 g | VP/Methacrylamide/ Vinyl Imidazole Copolymer |
| 1,90 g | Polyquaternium-46 |
| 2,00 g | Copolymer gemäß Beispiel H1 |
| 0,10 g | Parfumöl / etherisches Öl |
| 55,00 g | Alcohol |
| 39,00 g | Aqua dem. |

Gute Pumphaarsprays VOC 55 werden auch erhalten, wenn anstelle des Copolymers gemäß Beispiel H1 eines oder mehrere der Copolymere H 1 b, H2, H3, H3b, H3c, H4, H4b, H5, H6, H7 oder H8 verwendet werden.

Hautkosmetische Zusammensetzungen

Beispiel 17: Flüssiges Makeup

| | |
|---|---|
| Phase A | |
| 1,70 g | Glyceryl Stearate |
| 1,70 g | Cetyl Alcohol |
| 1,70 g | Ceteareth-6 |
| 1,70 g | Ceteareth-25 |
| 5,20 g | Caprylic/Capric Triglyceride |

(fortgesetzt)

| | |
|---|---|
| Phase A | |
| 5,20 g | Mineral Oil oder Luvitol® Lite (INCI Hydrogenated Polyisobuten) |
| Phase B | |
| q.s. | Konservierungsmittel |
| 4,30 g | Propylene Glycol |
| 2,50 g | Copolymer gemäß Beispiel H1 |
| 59,50 g | Aqua dem. |
| Phase C | |
| 0,10 g | Parfumöl / etherisches Öl |
| Phase D | |
| 2,00 g | Iron Oxides |
| 12,00 g | Titanium Dioxide |

Gute Flüssig-Make-ups werden auch erhalten, wenn anstelle des Copolymers gemäß Beispiel H1 eines oder mehrere der Copolymere H1b, H2, H3, H3b, H3c, H4, H4b, H5, H6, H7 oder H8 verwendet werden.

Beispiel 18: Eyeliner

| | |
|---|---|
| Phase A | |
| 40,60 g | dest. Wasser |
| 0,20 | Disodium EDTA |
| q.s. | Konservierungsmittel |
| Phase B | |
| 0,60 g | Xanthan Gum |
| 0,40 g | Veegum |
| 3,00 g | Butylene Glycol |
| 0,20 g | Polysorbate- 20 |
| Phase C | |
| 15,00 g | Iron oxide / Al Powder / Silica (z.B. Sicopearl® Fantastico Gold von BASF) |
| Phase D | |
| 10,00 g | Aqua dem. |
| 25,00 g | Festigerpolymer (z.B. Polyurethane-1 oder VP/Methacrylamide/ Vinyl Imidazole Copolymer, etc.) |
| 5,00 g | Copolymer gemäß Beispiel H1 |

Gute Eyeliner werden auch erhalten, wenn anstelle des Copolymers gemäß Beispiel H1 eines oder mehrere der Copolymere H1b, H2, H3, H3b, H3c, H4, H4b, H5, H6, H7 oder H8 verwendet werden.

Beispiel 19: Sonnenschutz-Gel

| | |
|---|---|
| Phase A | |
| 0,90 g | Copolymer gemäß Beispiel H1 |
| 8,00 g | Octyl Methoxycinnamate |
| 5,00 g | Octocrylene |

(fortgesetzt)

| | |
|---|---|
| Phase A | |
| 0,80 g | Octyl Triazone |
| 2,00 g | Butyl Methoxydibenzoylmethane |
| 2,00 g | Tocopheryl Acetate |
| 0,10 g | Parfumöl / etherisches Öl |
| Phase B | |
| 0,30 g | Acrylates/C10-30 Alkyl Acrylate Crosspolymer |
| 0,20 g | Carbomer |
| 5,00 g | Glycerin |
| 0,20 | Disodium EDTA |
| q.s. | Konservierungsmittel |
| 75,30 g | Aqua dem. |
| Phase C | |
| 0,20 g | Sodium Hydroxide |

Gute Sonnenschutzgele werden auch erhalten, wenn anstelle des Copolymers gemäß Beispiel H1 eines oder mehrere der Copolymere H1b, H2, H3, H3b, H3c, H4, H4b, H5, H6, H7 oder H8 verwendet werden.

Beispiel 20: Sonnenschutzemulsion mit $TiO_2$ und $ZnO_2$

| | |
|---|---|
| Phase A | |
| 1,00 g | PEG-7 Hydrogenated Castor Oil |
| 5,00 g | Copolymer gemäß Beispiel H1 |
| 2,00 g | PEG-45/Dodecyl Glycol Copolymer |
| 3,00 g | Isopropyl Myristate |
| 7,90 g | Jojoba (Buxus Chinensis) Oil |
| 4,00 g | Octyl Methoxycinnamate |
| 2,00 g | 4-Methylbenzylidene Camphor |
| 3,00 g | Titanium Dioxide, Dimethicone |
| 1,00 g | Dimethicone |
| 5,00 g | Zinc Oxide, Dimethicone |
| Phase B | |
| 0,20 | Disodium EDTA |
| 5,00 g | Glycerin |
| q.s. | Konservierungsmittel |
| 60,80 g | Aqua dem. |
| Phase C | |
| 0,10 g | Parfumöl / etherisches Öl |

Gute Sonnenschutzemulsionen werden auch erhalten, wenn anstelle des Copolymers gemäß Beispiel H1 eines oder mehrere der Copolymere H 1 b, H2, H3, H3b, H3c, H4, H4b, H5, H6, H7 oder H8 verwendet werden.

Beispiel 21: Gesichtswasser

| | |
|---|---|
| Phase A | |
| 3,00 g | Copolymer gemäß Beispiel H1 |
| 0,10 g | Parfumöl / etherisches Öl |
| 0,30 g | Bisabolol |
| Phase B | |
| 3,00 g | Glycerin |
| 1,00 g | Hydroxyethyl Cetyldimonium Phosphate |
| 5,00 g | Whitch Hazel (Hamamelis Virginiana) Distillate |
| 0,50 g | Panthenol |
| q.s. | Konservierungsmittel |
| 87,60 g | Aqua dem. |

Gute Gesichtswässer werden auch erhalten, wenn anstelle des Copolymers gemäß Beispiel H1 eines oder mehrere der Copolymere H1b, H2, H3, H3b, H3c, H4, H4b, H5, H6, H7 oder H8 verwendet werden.

Beispiel 22: Gesichtswaschpaste mit Peelingeffekt

| | |
|---|---|
| Phase A | |
| 73,00 g | Aqua dem. |
| 1,50 g | Carbomer |
| q.s. | Konservierungsmittel |
| Phase B | |
| q.s. | Parfümöl |
| 7,00 g | Potassium Cocoyl Hydrolyzed Protein |
| 4,00 g | Copolymer gemäß Beispiel H1 |
| Phase C | |
| 1,50 g | Triethanolamine |
| Phase D | |
| 13,00 g | Polyethylene (Luwax A™ von BASF) |

Gute Gesichtswaschpasten werden auch erhalten, wenn anstelle des Copolymers gemäß Beispiel H1 eines oder mehrere der Copolymere H 1 b, H2, H3, H3b, H3c, H4, H4b, H5, H6, H7 oder H8 verwendet werden.

Beispiel 23: Seife

| | |
|---|---|
| Phase A | |
| 25,00 g | Potassium Cocoate |
| 20,00 | Disodium Cocoamphodiacetate |
| 2,00 g | Lauramide DEA |
| 1,0 g | Glycol Stearate |
| 2,00 g | Copolymer gemäß Beispiel H1 |
| 50,00 g | Aqua dem. |
| q.s. | Citric Acid |

(fortgesetzt)

| | |
|---|---|
| Phase B | |
| q.s. | Konservierungsmittel |
| 0,10 g | Parfumöl / etherisches Öl |

Gute Seifen werden auch erhalten, wenn anstelle des Copolymers gemäß Beispiel H1 eines oder mehrere der Copolymere H1b, H2, H3, H3b, H3c, H4, H4b, H5, H6, H7 oder H8 verwendet werden.

Beispiel 24: Gesichtsreinigungsmilch Typ O/W

| | |
|---|---|
| Phase A | |
| 1,50 g | Ceteareth-6 |
| 1,50 g | Ceteareth-25 |
| 2,00 g | Glyceryl Stearate |
| 2,00 g | Cetyl Alcohol |
| 10,00 g | Mineral Oil |
| Phase B | |
| 5,00 g | Propylene Glycol |
| q.s. | Konservierungsmittel |
| 1,00 g | Copolymer gemäß Beispiel H1 |
| 66,30 g | Aqua dem. |
| Phase C | |
| 0,20 g | Carbomer |
| 10,00 g | Cetearyl Octanoate |
| Phase D | |
| 0,40 g | Tetrahydroxypropyl Ethylenediamine |
| Phase E | |
| 0,10 g | Parfumöl / etherisches Öl |
| 0,10 g | Bisabolol |

Gute Gesichtsreinigungsmilche werden auch erhalten, wenn anstelle des Copolymers gemäß Beispiel H1 eines oder mehrere der Copolymere H 1 b, H2, H3, H3b, H3c, H4, H4b, H5, H6, H7 oder H8 verwendet werden.

Beispiel 25: Transparente Seife

| | |
|---|---|
| 4,20 g | Sodium Hydroxide |
| 3,60 g | dest. Wasser |
| 10,00 g | Copolymer gemäß Beispiel H1 |
| 22,60 g | Propylene Glycol |
| 18,70 g | Glycerin |
| 5,20 g | Cocoamide DEA |
| 2,40 g | Cocamine Oxide |
| 4,20 g | Sodium Lauryl Sulfate |
| 7,30 g | Myristic Acid |
| 16,60 g | Stearic Acid |

(fortgesetzt)

| | |
|---|---|
| 5,20 g | Tocopherol |

Gute transparente Seifen werden auch erhalten, wenn anstelle des Copolymers gemäß Beispiel H1 eines oder mehrere der Copolymere H1b, H2, H3, H3b, H3c, H4, H4b, H5, H6, H7 oder H8 verwendet werden.

Beispiel 26: Rasierschaum

| | |
|---|---|
| 6,00 g | Ceteareth-25 |
| 5,00 g | Poloxamer 407 |
| 52,00 g | Aqua dem. |
| 1,00 g | Triethanolamine |
| 5,00 g | Propylene Glycol |
| 1,00 g | PEG-75 Lanolin Oil |
| 5,00 g | Copolymer gemäß Beispiel H1 |
| q.s. | Konservierungsmittel |
| 0,10 g | Parfumöl / etherisches Öl |
| 25,00 g | Sodium Laureth Sulfate |

Abfüllung: 90 Teile Wirksubstanz und 10 Teile Propan/Butan-Mischung 25:75.

Gute Rasierschäume werden auch erhalten, wenn anstelle des Copolymers gemäß Beispiel H1 eines oder mehrere der Copolymere H1b, H2, H3, H3b, H3c, H4, H4b, H5, H6, H7 oder H8 verwendet werden.

Beispiel 27: After Shave Balsam

| | |
|---|---|
| Phase A | |
| 0,25 g | Acrylates/C10-30 Alkyl Acrylate Crosspolymer |
| 1,50 g | Tocopheryl Acetate |
| 0,20 g | Bisabolol |
| 10,00 g | Caprylic/Capric Triglyceride |
| q.s. | Parfum |
| 1,00 g | Copolymer gemäß Beispiel H1 |
| Phase B | |
| 1,00 g | Panthenol |
| 15,00 g | Alcohol |
| 5,00 g | Glycerin |
| 0,05 g | Hydroxyethyl Cellulose |
| 1,90 g | Copolymer gemäß Beispiel H1 |
| 64,02 g | dest. Wasser |
| Phase C | |
| 0,08 g | Sodium Hydroxide |

Gute After-Shave-Balsams werden auch erhalten, wenn anstelle des Copolymers gemäß Beispiel H1 eines oder mehrere der Copolymere H1 b, H2, H3, H3b, H3c, H4, H4b, H5, H6, H7 oder H8 verwendet werden.

Beispiel 28: Pflegecreme

| | |
|---|---|
| Phase A | |
| 2,00 g | Ceteareth-6 |
| 2,00 g | Ceteareth-25 |
| 2,00 g | Cetearyl Alcohol |
| 3,00 g | Glyceryl Stearate SE |
| 5,00 g | Mineral Oil |
| 4,00 g | Jojoba (Buxus Chinensis) Oil |
| 3,00 g | Cetearyl Octanoate |
| 1,00 g | Dimethicone |
| 3,00 g | Mineral Oil, Lanolin Alcohol |
| Phase B | |
| 5,00 g | Propylene Glycol |
| 0,50 g | Veegum |
| 1,00 g | Panthenol |
| 1,70 g | Copolymer gemäß Beispiel H1 |
| 6,00 g | Polyquaternium-44 |
| q.s. | Konservierungsmittel |
| 60,80 g | Auqa dem. |
| Phase C | |
| q.s. | Parfum |

Gute Pflegecremes werden auch erhalten, wenn anstelle des Copolymers gemäß Beispiel H1 eines oder mehrere der Copolymere H1b, H2, H3, H3b, H3c, H4, H4b, H5, H6, H7 oder H8 verwendet werden.

Mund- und Zahnpflegezubereitungen

Beispiel 29: Zahnpaste

| | |
|---|---|
| Phase A | |
| 34,79 g | Aqua dem. |
| 3,00 g | Copolymer gemäß Beispiel H1 |
| 20,00 g | Glycerin |
| 0,76 g | Sodium Monofluorophosphate |
| Phase B | |
| 1,20 g | Sodium Carboxymethylcellulose |
| Phase C | |
| 0,80 g | Aromaöl |
| 0,06 g | Saccharin |
| q.s. | Konservierungsmittel |
| 0,05 g | Bisabolol |
| 1,00 g | Panthenol |
| 0,50 g | Tocopheryl Acetate |
| 2,80 g | Silica |

(fortgesetzt)

| | |
|---|---|
| Phase C | |
| 1,00 g | Sodium Lauryl Sulfate |
| 7,90 g | Dicalciumphosphate Anhydrate |
| 25,29 g | Dicalciumphosphate Dihydrate |
| 0,45 g | Titanium Dioxide |

Gute Zahnpasten werden auch erhalten, wenn anstelle des Copolymers gemäß Beispiel H1 eines oder mehrere der Copolymere H1b, H2, H3, H3b, H3c, H4, H4b, H5, H6, H7 oder H8 verwendet werden.

Beispiel 30: Mundwasser

| | |
|---|---|
| Phase A | |
| 2,00 g | Aromaöl |
| 4,50 g | Copolymer gemäß Beispiel H1 |
| 1,00 g | Bisabolol |
| 30,00 g | Alcohol |
| Phase B | |
| 0,20 g | Saccharin |
| 5,00 g | Glycerin |
| q.s. | Konservierungsmittel |
| 5,00 g | Poloxamer 407 |
| 52,30 g | Aqua dem. |

Gute Mundwässer werden auch erhalten, wenn anstelle des Copolymers gemäß Beispiel H1 eines oder mehrere der Copolymere H1b, H2, H3, H3b, H3c, H4, H4b, H5, H6, H7 oder H8 verwendet werden.

Beispiel 37: Prothesenhaftmittel

| | |
|---|---|
| Phase A | |
| 0,20 g | Bisabolol |
| 1,00 g | Beta-Carotene |
| q.s. | Aromaöl |
| 20,00 g | Cetearyl Octanoate |
| 5,00 g | Silica |
| 33,80 g | Mineral Oil |
| Phase B | |
| 5,00 g | Copolymer gemäß Beispiel H1 |
| 35,00 g | PVP (20%ige Lösung in Wasser) |

Gute Prothesenhaftmittel werden auch erhalten, wenn anstelle des Copolymers gemäß Beispiel H1 eines oder mehrere der Copolymere H1b, H2, H3, H3b, H3c, H4, H4b, H5, H6, H7 oder H8 verwendet werden.

Beispiel 38: Flüssige Seife

| | |
|---|---|
| 15,0 g | Kokosfettsäure, Kaliumsalz |
| 3,0 g | Kaliumoleat |
| 5,0 g | Luvitol®Lite (BASF) |
| 2,0 g | Polymer Vinylpyrrolidon/Stearylmethacrylat 70/30 Gew.-% (K-Wert 47; 1 % in Isopropanol) |
| 1,0 g | Glycerinstearat |
| 0,5 g | Copolymer gemäß Beispiel H1 |
| 2,0 g | Ethylenglycoldistearat |
| ad 100 | Spezifische Zusätze, Komplexierungsmittel, Duftstoffe, Wasser |

Gute Flüssigseifen werden auch erhalten, wenn anstelle des Copolymers gemäß Beispiel H1 eines oder mehrere der Copolymere H1b, H2, H3, H3b, H3c, H4, H4b, H5, H6, H7 oder H8 verwendet werden.

Beispiele 39-41: Conditionier Shampoo mit Perlglanz

Angaben in Gew.-%

| Zusatz | Bsp. 39 | Bsp.40 | Bsp. 41 |
|---|---|---|---|
| Copolymer gemäß Beispiel H1 | 0,5 | 0,5 | 0,5 |
| Natriumlaurethsulfat | 9,0 | 9,0 | 9,0 |
| Cocoamidopropylbetain | 2,5 | 2,5 | 2,5 |
| Benzophenon-3 | 1,5 | 0,5 | 1,00 |
| Perlglanzmittel | 2,0 | 2,0 | 2,0 |
| Luvitol Lite®(BASF) | 0,1 | 0,15 | 0,05 |
| Dinatrium EDTA | 0,1 | 0,2 | 0,15 |
| Konvervierungsmittel, Parfüm, Verdicker, pH-Einstellung und Lösungsvermittler | q.s. | q.s. | q.s. |
| Wasser | ad 100,0 | ad 100,0 | ad 100,0 |
| Der pH-Wert wird auf 6 eingestellt. | | | |

Gute Conditionier Shampoos mit Perlglanz werden auch erhalten, wenn anstelle des Copolymers gemäß Beispiel H1 eines oder mehrere der Copolymere H1b, H2, H3, H3b, H3c, H4, H4b, H5, H6, H7 oder H8 verwendet werden.

Beispiele 42-46: Formulierungen zum Duschen, Waschen, Baden

Angaben in Gew.-%

| Zusatz | Bsp. 42 | Bsp. 43 | Bsp. 44 | Bsp. 45 | Bsp. 46 |
|---|---|---|---|---|---|
| Texapon N 70 | 13,00 | 15,00 | 10,50 | 12,50 | 10,00 |
| Dehyton PK 45 | 7,50 | 7,00 | 5,00 | 5,50 | 10,00 |
| Cetiol HE | 2,00 | 2,50 | 3,50 | 5,00 | 2,30 |
| Parfüm | 0,10 | 0,10 | 0,10 | 0,10 | 0,10 |
| Luvitol®Lite (BASF) | 1,00 | 4,50 | 7,00 | 1,40 | 3,00 |
| D-Panthenol USP | 1,00 | 1,50 | 1,80 | 1,70 | 1,40 |
| Konservierungsmittel | 0,10 | 0,10 | 0,10 | 0,10 | 0,10 |
| Zitronensäure | 0,10 | 0,10 | 0,10 | 0,10 | 0,10 |
| Copolymer gemäß Beispiel H1 | 0,50 | 1,00 | 0,50 | 0,20 | 0,10 |

(fortgesetzt)

| Zusatz | Bsp. 42 | Bsp. 43 | Bsp. 44 | Bsp. 45 | Bsp. 46 |
|---|---|---|---|---|---|
| Natriumchlorid | 1,50 | 1,40 | 1,40 | 1,30 | 1,50 |
| Wasser dem. | ad 100 | ad 100 | ad 100 | ad 100 | ad 100 |

Gute Formulierungen zum Duschen, Waschen, Baden werden auch erhalten, wenn anstelle des Copolymers gemäß Beispiel H1 eines oder mehrere der Copolymere H 1 b, H2, H3, H3b, H3c, H4, H4b, H5, H6, H7 oder H8 verwendet werden.

**Patentansprüche**

1. Copolymer erhältlich durch Polymerisation von

a) wenigstens einem $\alpha,\beta$-ethylenisch ungesättigten Monomer a) der allgemeinen Formel I

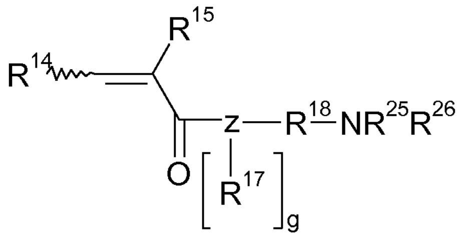

(I)

wobei

$R^{14}$ und $R^{15}$ unabhängig voneinander ausgewählt sind aus der Gruppe bestehend aus Wasserstoff, C1-C8 linear- oder verzweigtkettige Alkyl, Methoxy, Ethoxy, 2-Hydroxyethoxy, 2-Methoxyethoxy und 2-Ethoxyethyl,
$R^{17}$ Wasserstoff oder Methyl ist,
$R^{18}$ Alkylen oder Hydroxyalkylen mit 1 bis 24 C-Atomen, optional substituiert durch Alkyl, bevorzugt $C_2H_4$, $C_3H_6$, $C_4H_8$, $CH_2$-CH(OH)-$CH_2$ ist,
g 0 oder 1 ist,
Z Stickstoff wenn g = 1 oder Sauerstoff wenn g = 0 ist,
$R^{25}$ bzw. $R^{26}$ jeweils und unabhängig voneinander ausgewählt sind aus der Gruppe bestehend aus Wasserstoff, C1-C40 linear- oder verzweigtkettige Alkyl, Formyl, C1-C10 linear- oder verzweigtkettige Acyl, N,N-Dimethylaminoethyl, 2-Hydroxyethyl, 2-Methoxyethyl, 2-Ethoxyethyl, Hydroxypropyl, Methoxypropyl, Ethoxypropyl oder Benzyl,

wobei wenigstens 60 Mol-% von a) wenigstens ein quartäres Stickstoffatom aufweisen,
b) wenigstens einem $\alpha,\beta$-ethylenisch ungesättigten Monomer b) der allgemeinen Formel II

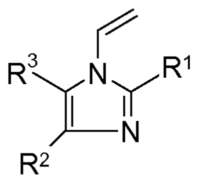

(II)

wobei $R^1$ bis $R^3$ unabhängig voneinander Wasserstoff, $C_1$-$C_4$-Alkyl oder Phenyl bedeuten;
c) wenigstens einem $\alpha,\beta$-ethylenisch ungesättigten Monomer c) der allgemeinen Formel III

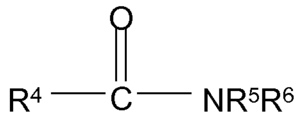

(III)

wobei

einer der Reste $R^4$ bis $R^6$ für eine Gruppe der Formel $CH_2$=$CR^7$- mit $R^7$ = H oder $C_1$-$C_4$-Alkyl steht und die übrigen Reste $R^4$ bis $R^6$ unabhängig voneinander für H, Alkyl, Cycloalkyl, Heterocycloalkyl, Aryl oder Hetaryl stehen,
wobei $R^4$ und $R^5$ gemeinsam mit der Amidgruppe, an die sie gebunden sind, auch für ein Lactam mit 5 bis 8 Ringatomen stehen können,
wobei $R^5$ und $R^6$ gemeinsam mit dem Stickstoffatom, an das sie gebunden sind, auch für einen fünf- bis siebengliedrigen Heterocyclus stehen können,

d) gegebenenfalls wenigstens einem weiteren radikalisch polymerisierbaren, von a), b) und c) verschiedenen Monomer d).

**2.** Copolymer nach Anspruch 1, wobei wenigstens ein Monomer a) ausgewählt ist aus der Gruppe bestehend aus N,N-Dimethylaminoalkyl(meth)acrylamiden, N,N-Dimethylaminoalkyl(meth)acrylestern und deren Mischungen.

**3.** Copolymer nach Anspruch 1 oder 2, wobei wenigstens ein Monomer a) ausgewählt ist aus der Gruppe bestehend aus N-[3-(dimethylamino)propyl]-methacrylamid, N,N-Dimethylaminoethylmethacrylat und deren Mischungen.

**4.** Copolymer nach einem der Ansprüche 1 bis 3, wobei wenigstens 90% der Stickstoffatome von Monomer a) in quaternisierter Form vorliegen.

**5.** Copolymer nach einem der Ansprüche 1 bis 4, wobei wenigstens ein Monomer b) N-Vinylimidazol ist.

**6.** Copolymer nach einem der Ansprüche 1 bis 5, wobei wenigstens ein Monomer c) ausgewählt ist aus der Gruppe bestehend aus N-Vinylpyrrolidon, N-Vinylcaprolactam und deren Mischungen.

**7.** Copolymer nach einem der Ansprüche 1 bis 6 erhältlich durch Polymerisation von

a) 60-90 Gew.-% Monomer a)
b) 5-35 Gew.-% Monomer b)
c) 5-35 Gew.-% Monomer c)
d) 0 bis 20 Gew.-% Monomer d),

jeweils bezogen auf das Gesamtgewicht der zur Polymerisation eingesetzten Monomere,
mit der Maßgabe, dass die Summe der Mengen der Monomere a) bis d) 100 Gew.-% ergibt.

**8.** Copolymer nach einem der Ansprüche 1 bis 7 erhältlich durch Polymerisation von

a) 70-80 Gew.-% Monomer a)
b) 10-20 Gew.-% Monomer b)
c) 10-20 Gew.-% Monomer c)
d) 0 bis 10 Gew.-% Monomer d)

jeweils bezogen auf die Gesamtmenge der Monomere a) bis d),
mit der Maßgabe, dass die Summe der Mengen der Monomere a) bis d) 100 Gew.-% ergibt.

**9.** Copolymer nach einem der Ansprüche 1 bis 8, wobei Monomer d) eine anionische oder anionogene Verbindung ist oder umfasst.

**10.** Copolymer nach einem der Ansprüche 1 bis 9, wobei die Komponente d) Methacrylsäure ist oder umfasst.

**11.** Kosmetisches oder pharmazeutisches Mittel, enthaltend

A) wenigstens ein Copolymer wie in einem der Ansprüche 1 bis 10 definiert und
B) wenigstens einen kosmetisch akzeptablen Träger.

**12.** Mittel nach Anspruch 11, wobei die Komponente B) ausgewählt ist unter

i) Wasser,
ii) wassermischbaren organischen Lösungsmitteln, vorzugsweise $C_2$-$C_4$-Alkanolen, insbesondere Ethanol,
iii) Ölen, Fetten, Wachsen,
iv) von iii) verschiedenen Estern von $C_6$-$C_{30}$-Monocarbonsäuren mit ein-, zwei- oder dreiwertigen Alkoholen,
v) gesättigten acyclischen und cyclischen Kohlenwasserstoffen,
iv) Fettsäuren,
vii) Fettalkoholen,
viii) Treibgasen

und Mischungen davon.

**13.** Mittel nach einem der Ansprüche 11 oder 12, enthaltend wenigstens einen von den Komponenten A) und B) verschiedenen Zusatzstoff, der ausgewählt ist unter kosmetisch aktiven Wirkstoffen, Emulgatoren, Tensiden, Konservierungsmitteln, Parfümölen, Verdickern, Haarpolymeren, Haar- und Hautconditionern, Pfropfpolymeren, wasserlöslichen oder dispergierbaren silikonhaltigen Polymeren, Lichtschutzmitteln, Bleichmitteln, Gelbildnern, Pflegemitteln, Färbemitteln, Tönungsmitteln, Bräunungsmitteln, Farbstoffen, Pigmenten, Konsistenzgebern, Feuchthaltemitteln, Rückfettern, Collagen, Eiweißhydrolysaten, Lipiden, Antioxidantien, Entschäumern, Antistatika, Emollienzien und Weichmachern.

**14.** Mittel nach einem der Ansprüche 11 bis 13 in Form eines Sprays, Gels, Schaums, einer Mousse, Salbe, Creme, Emulsion, Suspension, Lotion, Milch oder Paste.

**15.** Haarspülung oder Haarshampoo enthaltend ein Copolymer wie in einem der Ansprüche 1 bis 10 definiert.

**16.** Verwendung eines Copolymers, wie in einem der Ansprüche 1 bis 10 definiert, in Hautreinigungsmitteln, Mitteln zur Pflege und zum Schutz der Haut, Nagelpflegemitteln, Zubereitungen für die dekorative Kosmetik und Haarbehandlungsmitteln.

**17.** Verwendung nach Anspruch 16 in Haarbehandlungsmitteln als Festiger und/oder als Conditioner.

**18.** Verwendung nach Anspruch 17, wobei das Mittel in Form eines Haargels, Shampoos, Schaumfestigers, Haarwassers, Haarsprays oder Haarschaums vorliegt.

**19.** Verwendung eines Copolymers, wie in einem der Ansprüche 1 bis 10 definiert, als Hilfsmittel in der Pharmazie, bevorzugt als oder in Beschichtungsmittel(n) für feste Arzneiformen, zur Modifizierung rheologischer Eigenschaften, als oberflächenaktive Verbindung, als oder in Klebemittel(n) sowie als oder in Beschichtungsmittel(n) für die Textil-, Papier-, Druck- und Lederindustrie.

## Claims

**1.** A copolymer obtainable by polymerization of

a) at least one $\alpha,\beta$-ethylenically unsaturated monomer a) of the general formula I

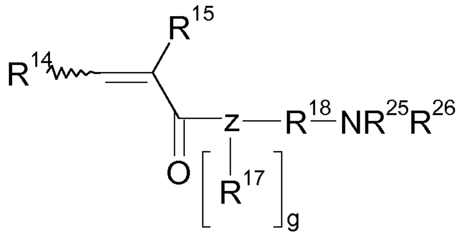

(I)

where
$R^{14}$ and $R^{15}$, independently of one another, are selected from the group consisting of hydrogen, C1-C8 linear- or branched-chain alkyl, methoxy, ethoxy, 2-hydroxyethoxy, 2-methoxyethoxy and 2-ethoxyethyl, $R^{17}$ is hydrogen or methyl, $R^{18}$ is alkylene or hydroxyalkylene having 1 to 24 carbon atoms, optionally substituted by alkyl, preferably $C_2H_4$, $C_3H_6$, $C_4H_8$, $CH_2$-CH (OH)-$CH_2$, g is 0 or 1, Z is nitrogen when g = 1 or oxygen when g = 0, $R^{25}$ and $R^{26}$ are in each case and independently of one another selected from the group consisting of hydrogen, C1-C40 linear- or branched-chain alkyl, formyl, C1-C10 linear- or branched-chain acyl, N,N-dimethylaminoethyl, 2-hydroxyethyl, 2-methoxyethyl, 2-ethoxyethyl, hydroxypropyl, methoxypropyl, ethoxypropyl or benzyl,
where at least 60 mol% of a) have at least one quaternary nitrogen atom,
b) at least one $\alpha,\beta$-ethylenically unsaturated monomer b) of the general formula II

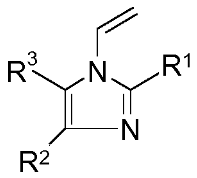 (II)

where $R^1$ to $R^3$, independently of one another, are hydrogen, $C_1$-$C_4$-alkyl or phenyl;
c) at least one $\alpha,\beta$-ethylenically unsaturated monomer c) of the general formula III

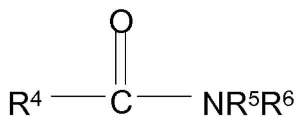 (III)

where

one of the radicals $R^4$ to $R^6$ is a group of the formula $CH_2$=$CR^7$- where $R^7$ = H or $C_1$-$C_4$-alkyl and the other radicals $R^4$ to $R^6$, independently of one another, are H, alkyl, cycloalkyl, heterocycloalkyl, aryl or hetaryl, where $R^4$ and $R^5$, together with the amide group to which they are bonded may also be a lactam having 5 to 8 ring atoms,
where $R^5$ and $R^6$, together with the nitrogen atom to which they are bonded, may also be a five- to seven-membered heterocycle,

d) optionally at least one further free-radically polymerizable monomer d) different from a), b) and c).

2. The copolymer according to claim 1, where at least one monomer a) is selected from the group consisting of N,N-dimethylaminoalkyl(meth)acrylamides, N,N-dimethylaminoalkyl (meth)acrylic esters and mixtures thereof.

3. The copolymer according to claim 1 or 2, where at least one monomer a) is selected from the group consisting of N-[3-(dimethylamino)propyl]meth-acrylamide, N,N-dimethylaminoethyl methacrylate and mixtures thereof.

4. The copolymer according to any of claims 1 to 3, where at least 90% of the nitrogen atoms of monomer a) are present in quaternized form.

5. The copolymer according to any of claims 1 to 4, where at least one monomer b) is N-vinylimidazole.

6. The copolymer according to any of claims 1 to 5, where at least one monomer c) is selected from the group consisting of N-vinylpyrrolidone, N-vinylcaprolactam and mixtures thereof.

7. The copolymer according to any of claims 1 to 6, obtainable by polymerization of

a) 60-90% by weight of monomer a)
b) 5-35% by weight of monomer b)
c) 5-35% by weight of monomer c)
d) 0 to 20% by weight of monomer d), in each case based on the total weight of the monomers used for the polymerization, with the proviso that the sum of the amounts of the monomers a) to d) is 100% by weight.

**8.** The copolymer according to any of claims 1 to 7, obtainable by polymerization of

a) 70-80% by weight of monomer a)
b) 10-20% by weight of monomer b)
c) 10-20% by weight of monomer c)
d) 0 to 10% by weight of monomer d) in each case based on the total amount of the monomers a) to d), with the proviso that the sum of the amounts of the monomers a) to d) is 100% by weight.

**9.** The copolymer according to any of claims 1 to 8, where monomer d) is or comprises an anionic or anionogenic compound.

**10.** The copolymer according to any of claims 1 to 9, where component d) is or comprises methacrylic acid.

**11.** A cosmetic or pharmaceutical composition comprising

A) at least one copolymer as defined in any of claims 1 to 10 and
B) at least one cosmetically acceptable carrier.

**12.** The composition according to claim 11, where component B) is selected from

i) water,
ii) water-miscible organic solvents, preferably $C_2$-$C_4$-alkanols, in particular ethanol,
iii) oils, fats, waxes,
iv) esters of $C_6$-$C_{30}$-monocarboxylic acids with mono-, di- or trihydric alcohols that are different from iii),
v) saturated acyclic and cyclic hydrocarbons,
iv) fatty acids,
vii) fatty alcohols,
viii) propellant gases

and mixtures thereof.

**13.** The composition according to any of claims 11 or 12, comprising at least one additive different from components A) and B) which is selected from cosmetically active ingredients, emulsifiers, surfactants, preservatives, perfume oils, thickeners, hair polymers, hair and skin conditioners, graft polymers, water-soluble or dispersible silicone-containing polymers, photoprotective agents, bleaches, gel formers, care agents, colorants, tinting agents, tanning agents, dyes, pigments, consistency regulators, humectants, refatting agents, collagen, protein hydrolyzates, lipids, antioxidants, antifoams, antistats, emollients and softeners.

**14.** The composition according to any of claims 11 to 13 in the form of a spray, gel, foam, mousse, ointment, cream, emulsion, suspension, lotion, milk or paste.

**15.** A hair rinse or hair shampoo comprising a copolymer as defined in any of claims 1 to 10.

**16.** The use of a copolymer, as defined in any of claims 1 to 10, in skin cleansing compositions, compositions for the care and protection of the skin, nail care compositions, preparations for decorative cosmetics and hair treatment compositions.

**17.** The use according to claim 16 in hair treatment compositions as setting agent and/or as conditioner.

**18.** The use according to claim 17, where the composition is in the form of a hair gel, shampoo, setting foam, hair tonic, hairspray or hair foam.

**19.** The use of a copolymer, as defined in any of claims 1 to 10, as auxiliary in pharmacy, preferably as or in (a) coating composition(s) for solid drug forms, for the modification of rheological properties, as surface-active compound, as or in (an) adhesive(s), and as or in (a) coating composition(s) for the textile, paper, printing and leather industries.

## Revendications

**1.** Copolymère pouvant être obtenu par polymérisation de

a) au moins un monomère a) à insaturation α,β-éthylénique de formule générale I

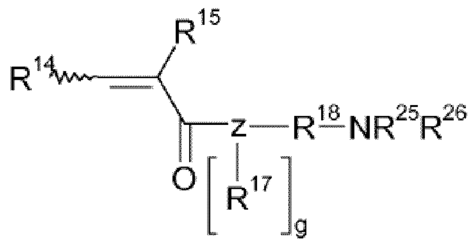

(I)

dans laquelle

$R^{14}$ et $R^{15}$ sont choisis indépendamment l'un de l'autre dans le groupe constitué par un atome d'hydrogène, un groupe alkyle en $C_1$-$C_8$ à chaîne linéaire ou ramifiée, méthoxy, éthoxy, 2-hydroxyéthoxy, 2-méthoxyéthoxy et 2-hydroxy-éthyle,
$R^{17}$ représente un atome d'hydrogène ou un groupe méthyle,
$R^{18}$ représente un groupe alkylène ou hydroxy-alkylène ayant de 1 à 24 atomes de carbone, en option substitué par alkyle, de préférence $C_2H_4$, $C_3H_6$, $C_4H_8$, $CH_2$-CH(OH)-$CH_2$, g est 0 ou 1,
Z représente un atome d'azote lorsque g est égal à 1 ou un atome d'oxygène lorsque g est égal à 0,
$R^{25}$ et $R^{26}$ sont choisis chacun et indépendamment l'un de l'autre dans le groupe constitué par un atome d'hydrogène, un groupe alkyle en $C_1$-$C_{40}$ à chaîne linéaire ou ramifiée, formyle, acyle en $C_1$-$C_{10}$ à chaîne linéaire ou ramifiée, N,N-diméthylaminoéthyle, 2-hydroxyéthyle, 2-méthoxyéthyle, 2-éthoxyéthyle, hydroxypropyle, méthoxypropyle, éthoxypropyle ou benzyle,

au moins 60 % en moles de a) comportant au moins un atome d'azote quaternaire,
b) au moins un monomère b) à insaturation α,β-éthylénique de formule générale II

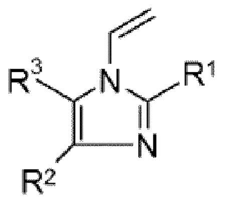

(II)

dans laquelle $R^1$ à $R^3$ représentent chacun indépendamment un atome d'hydrogène, un groupe alkyle en $C_1$-$C_4$ ou phényle ;
c) au moins un monomère c) à insaturation α,β-éthylénique de formule générale III

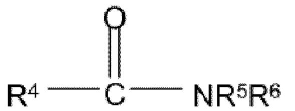

(III)

dans laquelle l'un des radicaux $R^4$ à $R^6$ représente un groupe de formule $CH_2$=$CR^7$- où $R^7$ représente H ou un groupe alkyle en $C_1$-$C_4$ et les radicaux $R^4$ à $R^6$ restants représentent indépendamment l'un de l'autre H, un groupe alkyle, cycloalkyle, hétérocycloalkyle, aryle ou hétéroaryle,
$R^4$ et $R^5$ pouvant également représenter ensemble, avec le groupe amido auquel ils sont liés, un lactame ayant de 5 à 8 atomes formant le cycle,
$R^4$ et $R^5$ pouvant également représenter ensemble, avec l'atome d'azote auquel ils sont liés, un hétérocycle à cinq à sept chaînons,
d) éventuellement au moins un autre monomère d) différent de a), b) et c), apte à la polymérisation radicalaire.

**2.** Copolymère selon la revendication 1, dans lequel au moins un monomère a) est choisi dans le groupe constitué par des N,N-diméthylaminoalkyl(méth)-acrylamides, des (méth)acrylates de N,N-diméthylamino-alkyle et leurs mélanges.

**3.** Copolymère selon la revendication 1 ou 2, dans lequel au moins un monomère a) est choisi dans le groupe constitué par le N-[3-(diméthylamino)propyl]-méthacrylamide, le méthacrylate de N,N-diméthylamino-éthyle et leurs mélanges.

**4.** Copolymère selon l'une quelconque des revendications 1 à 3, dans lequel au moins 90 % des atomes d'azote du monomère a) se trouvent sous forme quaternisée.

**5.** Copolymère selon l'une quelconque des revendications 1 à 4, dans lequel au moins un monomère b) est le N-vinylimidazole.

**6.** Copolymère selon l'une quelconque des revendications 1 à 5, dans lequel au moins un monomère c) est choisi dans le groupe constitué par la N-vinylpyrrolidone, le N-vinylcaprolactame et leurs mélanges.

**7.** Copolymère selon l'une quelconque des revendications 1 à 6, pouvant être obtenu par polymérisation de

a) 60-90 % en poids de monomère a)
b) 5-35 % en poids de monomère b)
c) 5-35 % en poids de monomère c)
d) 0 à 20 % en poids de monomère d),

chaque fois par rapport au poids total des monomères utilisés dans la polymérisation, étant entendu que la somme des quantités des monomères a) à d) est égale à 100 % en poids.

**8.** Copolymère selon l'une quelconque des revendications 1 à 7, pouvant être obtenu par polymérisation de

a) 70-80 % en poids de monomère a)
b) 10-20 % en poids de monomère b)
c) 10-20 % en poids de monomère c)
d) 0 à 10 % en poids de monomère d),

chaque fois par rapport à la quantité totale des monomères a) à d),
étant entendu que la somme des quantités des monomères a) à d) est égale à 100 % en poids.

**9.** Copolymère selon l'une quelconque des revendications 1 à 8, dans lequel le monomère d) est ou comprend un composé anionique ou anionogène.

**10.** Copolymère selon l'une quelconque des revendications 1 à 9, dans lequel le composant d) est ou comprend l'acide méthacrylique.

**11.** Produit cosmétique ou pharmaceutique, contenant

A) au moins un copolymère tel que défini dans l'une quelconque des revendications 1 à 10 et
B) au moins un véhicule cosmétiquement acceptable.

**12.** Produit selon la revendication 11, dans lequel le composant B) est choisi parmi

i) l'eau,
ii) des solvants organiques miscibles à l'eau, de préférence des alcanols en $C_2$-$C_4$, en particulier l'éthanol,
iii) des huiles, des graisses, des cires,
iv) des esters d'acides monocarboxyliques en $C_6$-$C_{30}$ avec des alcools mono-, di- ou trihydriques, différents de iii),
v) des hydrocarbures saturés acycliques et cycliques,
vi) des acides gras,
vii) des alcools gras,
viii) des gaz propulseurs

et des mélanges de ceux-ci.

13. Produit selon l'une quelconque des revendications 11 et 12, contenant au moins un additif différent des composants A) et B), qui est choisi parmi des substances cosmétiquement actives, des émulsifiants, des tensioactifs, des conservateurs, des huiles essentielles, des épaississants, des polymères pour cheveux, des conditionneurs pour les cheveux et pour la peau, des polymères greffés, des polymères contenant des silicones, dispersables ou solubles dans l'eau, des photoprotecteurs, agents décolorants, gélifiants, agents de soin, agents colorants, agents de nuançage, agents bronzants, colorants, pigments, agents de consistance, humectants, agents de regraissage, le collagène, des hydrolysats de protéines, des lipides, des antioxydants, antimousses, agents antistatiques, émollients et assouplissants.

14. Produit selon l'une quelconque des revendications 11 à 13, sous forme d'une composition à pulvériser en aérosol, d'un gel, de mousse, d'une mousse, d'une pommade, d'une crème, d'une émulsion, d'une suspension, d'une lotion, d'un lait ou d'une pâte.

15. Shampooing capillaire ou après-shampooing, contenant un copolymère tel que défini dans l'une quelconque des revendications 1 à 10.

16. Utilisation d'un copolymère tel que défini dans l'une quelconque des revendications 1 à 10, dans des produits pour le nettoyage de la peau, des produits pour le soin et pour la protection de la peau, des produits pour le soin des ongles, des préparations pour la cosmétique de maquillage et des produits de traitement capillaire.

17. Utilisation selon la revendication 16, dans des produits de traitement capillaire, en tant que fixateur et/ou en tant que conditionneur.

18. Utilisation selon la revendication 17, dans laquelle le produit se trouve sous forme d'un gel capillaire, d'un shampooing, d'un fixateur en mousse, d'une lotion capillaire, d'une laque pour cheveux ou d'une mousse coiffante.

19. Utilisation d'un copolymère tel que défini dans l'une quelconque des revendications 1 à 10, comme adjuvant en pharmacie, de préférence en tant qu'agent d'enrobage ou dans des compositions d'enrobage pour formes pharmaceutiques solides, pour la modification de propriétés rhéologiques, en tant que composé tensioactif, ou en tant qu'adhésif ou dans des adhésifs ainsi qu'en tant qu'agent de revêtement ou dans des compositions de revêtement pour les industries textile, du papier, de l'impression et du cuir.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 0042985 A
- DE 10241296
- WO 2005005497 A
- WO 06097514 A
- WO 2006106140 A
- US 6998113 B
- US 5935561 A
- WO 2006106114 A


### In der Beschreibung aufgeführte Nicht-Patentliteratur


- **KARL-HEINZ SCHRADER.** Grundlagen und Rezepturen der Kosmetika. 319-355
- Kosmetik und Hygiene von Kopf bis Fuß. Wiley-VCH, 2004, 131-134
- Kosmetik und Hygiene von Kopf bis Fuß. Wiley-VCH, 2004, 235-236
- Kosmetik und Hygiene von Kopf bis Fuß. Wiley-VCH, 2004, 123-128
- Kosmetik und Hygiene von Kopf bis Fuß. Wiley-VCH, 2004, 112-122
- Kosmetik und Hygiene von Kopf bis Fuß. Wiley-VCH, 2004, 128-134
- **FIKENTSCHER.** *Cellulosechemie,* 1932, vol. 13, 58-64